# FASCICULE DE BREVET EUROPEEN

(11) **EP 2 547 694 B1**
(45) Date de publication et mention de la délivrance du brevet: **17.08.2016**
(21) Numéro de dépôt: 11715936.8
(22) Date de dépôt: 17.03.2011
(51) Int. Cl.: C07K 7/08, C12N 15/62

(54) **NOUVEAU PEPTIDE SIGNAL, ET SON UTILISATION POUR LA PRODUCTION DE PROTEINES RECOMBINANTES**
NEUES SIGNALPEPTID UND VERWENDUNG ZUR HERSTELLUNG VON REKOMBINANTEN PROTEINEN
NOVEL SIGNAL PEPTIDE, AND USE THEREOF FOR PRODUCING RECOMBINANT PROTEINS

(30) Priorité: 17.03.2010 FR 1051905
(43) Date de publication de la demande: 23.01.2013
(73) Titulaire: Laboratoire Français du Fractionnement et des Biotechnologies, 91940 Les Ulis (FR)
(72) Inventeur: FONTAYNE, Alexandre, 59110 La Madeleine (FR)
(74) Mandataire: Grosset-Fournier, Chantal Catherine
(86) Numéro de dépôt international: PCT/FR2011/050544
(87) Numéro de publication internationale: WO 2011/114063

(56) Documents cités:
- US-A1- 2004 229 301
- US-B1- 7 008 623

## Description

La présente invention concerne un peptide signal, et son utilisation pour la production de protéines recombinantes dans les systèmes d'expression eucaryotes.

Le peptide signal (PS) est un segment de 12 à 30 acides aminés situé à l'extrémité N-terminale d'une protéine servant à adresser celle-ci à un compartiment cellulaire (organite) particulier ou permettant son adressage au milieu extracellulaire.

Le peptide signal induit le passage dans le Réticulum Endoplasmique Rugueux (RER) où la protéine va être maturée. Ainsi au cours de la synthèse de la protéine, une « particule de reconnaissance du signal » (SRP) se fixe à la fois au peptide signal (liaison récepteur-ligand) et au ribosome duquel le peptide signal émerge. L'ensemble SRP / ribosome est ensuite recruté sur la surface du réticulum endoplasmique par un récepteur membranaire, le récepteur du SRP, lui-même lié au canal de translocation (PCC). Au cours de cet événement la synthèse de la protéine est suspendue car le SRP bloque l'accès des facteurs d'élongation, eEF-1 et eEF-2. Ce recrutement est suivi par une hydrolyse du GTP, en GDP et Pi, qui libère la particule SRP.

La protéine naissante, au fur et à mesure de sa croissance, emprunte le canal pour passer entièrement dans lumière du réticulum. La nature hydrophobe du peptide signal (hélice-α) permet également une interaction avec les phospholipides de la membrane.

Lors de leur synthèse, les protéines destinées à être sécrétées conservent leur peptide signal qui persiste sous forme d'hélice-α comme site d'insertion dans la membrane, permettant la prise en charge de la chaîne polypeptidique en cours de synthèse par les protéines chaperonnes comme BIP pour assurer son repliement. Les modifications post-traductionnelles comme la glycosylation débutent dans le RE et sont achevées dans le Golgi, où les glycannes sont maturés.

L'extrémité N-terminale d'un peptide signal est répartie en 3 régions, à savoir la région n, la région h et la région c, avec chacune une longueur et des propriétés différentes. La région n se situe à l'extrémité N-terminale du peptide signal, tandis que la région c est à l'extrémité C-terminale du peptide signal. La région h se situe entre la région n et la région c. Les régions n et h constituent le coeur du peptide signal qui conditionne l'adressage tandis que la région c conditionne la coupure par une peptidase. Il est connu que la région n est peu riche en arginine, alors que la région h est une région hydrophobe. Il est aussi connu que les résidus dans la région c sont plutôt des résidus petits et non polaires.

Lors de l'expression d'une protéine recombinante dans un système eucaryote, la présence ou non de peptide signal, ou le choix de celui-ci peut influencer directement la productivité de ladite protéine recombinante, autrement dit la quantité de protéine sécrétée par les cellules contenant un transgène rapportée par unité de volume.

Par exemple, certaines protéines hétérologues sont toxiques pour la survie ou la croissance des cellules hôtes, certaines d'autres ne le sont pas, mais inhibent la croissance des cellules hôtes. Dans ces cas, la sécrétion d'une telle protéine toxique vers le milieu extracellulaire, permet d'éviter la toxicité ou l'inhibition de croissance liée à l'expression de la protéine recombinante hétérologue.

D'un point de vue de production industrielle, une protéine recombinante capable d'être sécrétée dans le milieu extra-cellulaire est beaucoup plus avantageuse par rapport à une protéine recombinante accumulée dans la cellule hôte, car le procédé de purification d'une protéine sécrétée est généralement plus simple que celui d'une protéine qui s'est accumulée dans une cellule. De plus, ceci permet de s'affranchir de la co-purification de protéines immatures pouvant interférer ou réduire l'activité de la protéine d'intérêt.

En outre, étant donné que tous les peptides signaux n'ont pas le même pouvoir sécrétoire, le peptide signal endogène d'une protéine capable d'être sécrétée n'est pas toujours assez efficace pour la production industrielle de la protéine en question.

Certains peptides signaux ont déjà été conçus pour l'expression de protéines recombinantes dans certains systèmes d'expression. Le brevet EP 0 329 127 décrit un peptide signal destiné à l'expression de protéines recombinantes dans des cellules de levures. Le brevet EP 0 356 335 décrit un peptide signal bactérien pour améliorer la production périplasmique de polypeptides hétérologues dans des bactéries.

Toutefois, ces peptides signaux ne permettent pas d'optimiser l'expression de protéines recombinantes dans les lignées cellulaires eucaryotes supérieures incluant les cellules de mammifère, un système d'expression souvent incontournable pour la production de nombreuses protéines recombinantes thérapeutiques, telles que des anticorps.

Par conséquent, il ressort qu'il y a une grande nécessité de mettre à disposition un peptide signal universel, permettant d'améliorer l'expression et la sécrétion de protéines recombinantes dans une lignée cellulaire eucaryote supérieure.

Le document US 2004/229301 décrit le peptide signal MAWVWTLLFLMAAAQSAQA.

La présente invention repose sur une constatation inattendue faite par les Inventeurs, lors d'une étude sur la capacité de sécrétion d'une part d'une chaîne légère d'un anticorps liée aux différents peptides signaux conçus par les Inventeurs, et d'autre part sur une IgG entière. En effet, certains peptides signaux permettent d'augmenter significativement la sécrétion d'une chaîne légère d'immunoglobuline ou d'une immunoglobuline entière.

La présente divulgation est ainsi illustrée par l'utilisation d'un peptide signal pour la production et la sécrétion d'un polypeptide recombinant d'intérêt dans un système d'expression, la quantité du polypeptide secrété à l'aide dudit peptide signal étant au moins égale à la quantité dudit polypeptide secrété à l'aide de son peptide signal naturel, l'activité biologique du polypeptide d'intérêt étant conservée par rapport à celle du polypeptide d'intérêt produit par son peptide signal naturel, ledit peptide signal comprenant au moins 12 acides aminés de formule (I) :

(X1)ᵢX2X3X4SX5X6X7,

dans laquelle : (inclut les trois peptides)
- X1 est un peptide contenant de 3 à 6 acides aminés, i égal à 0 ou 1,
- X2 est un peptide contenant de 3 à 9 acides aminés hydrophobes,
- X3 est un peptide contenant de 3 à 5 acides aminés, ledit peptide comprenant au moins 3 leucines, contigües ou non,
- X4 est un peptide contenant de 2 à 5 acides aminés choisis parmi Ala, Thr, Ser, Gln, Ile, Met,
- X5 est Ala ou Val,
- X6 est Gln, Asn ou His,
- X7 est Ala ou Cys,
sous réserve que :
- lorsque ledit peptide signal est issu d'un précurseur naturel d'une protéine déterminée, ledit polypeptide d'intérêt est différent de ladite protéine, et
- **lorsque le peptide signal est MAWVWTLLFLMAAAQSAQA, ledit polypeptide d'intérêt n'est pas un anticorps anti-CD23,**
   n'importe quel polypeptide d'intérêt pouvant être lié à l'un desdits peptides signaux pour être sécrété dans un milieu extracellulaire.

Dans un premier aspect, l'invention concerne ainsi l'utilisation d'un peptide signal pour la production d'un polypeptide recombinant d'intérêt dans un système d'expression, ledit peptide signal comprenant au moins 12 acides aminés de formule (I) :

(X1)iX2X3X4SX5X6X7,

dans laquelle :
- X1 est un peptide contenant de 3 à 6 acides aminés, i égal à 0 ou 1,
- X2 est un peptide contenant de 3 à 9 acides aminés hydrophobes choisis parmi Gly, Ala, Val, Ile, Pro, Phe, Trp,
- X3 est un peptide contenant de 3 à 5 acides aminés, ledit peptide comprenant au moins 3 leucines, contigües,
- X4 est un peptide contenant de 2 à 5 acides aminés choisis parmi Ala, Thr, Ser, Gln, Ile, Met,
- X5 est Ala ou Val,
- X6 est Gln, Asn ou His,
- X7 est Ala ou Cys,
sous réserve que lorsque ledit peptide signal est issu d'un précurseur naturel d'une protéine déterminée, ledit polypeptide d'intérêt est différent de ladite protéine, n'importe quel polypeptide d'intérêt pouvant être lié à l'un desdits peptides signaux pour être sécrété dans le milieu extracellulaire.

Par « peptide signal », on entend une chaîne peptidique d'environ de 10 à 30 acides aminés, située au sein d'une chaîne polypeptidique, +servant à adresser cette dernière à un compartiment cellulaire (organite) particulier ou permettant son adressage au milieu extracellulaire.

Ledit peptide signal selon l'invention se situe, de préférence, à l'extrémité N-terminale du polypeptide d'intérêt.

L'utilisation d'un peptide signal permet d'augmenter le taux de sécrétion d'un polypeptide d'intérêt. Il est à noter que le peptide signal utilisé dans l'invention est différent du peptide signal naturellement associé à une protéine déterminée.

On entend par « polypeptide recombinant d'intérêt » un polypeptide produit *in vitro* par la machinerie transcriptionnelle et traductionnelle d'une cellule en culture, ou plus généralement produit par des cellules vivantes (*in vitro* ou *in vivo)* ayant reçues un ADN exogène codant pour ledit polypeptide.

Ledit peptide signal permet de produire un polypeptide recombinant d'intérêt secrété en quantité au moins égale à la quantité dudit polypeptide secrété à l'aide de son peptide signal naturel.

De plus, un tel polypeptide d'intérêt produit à l'aide d'un peptide signal selon l'invention conserve l'activité biologique du polypeptide d'intérêt produit par son peptide signal naturel.

Un peptide signal selon l'invention peut permettre de produire un polypeptide recombinant d'intérêt dans un système d'expression *in vitro,* par exemple dans une culture cellulaire.

Un peptide signal selon l'invention peut permettre aussi de produire un polypeptide recombinant d'intérêt dans un système d'expression *in vivo,* par exemple dans des animaux transgéniques, tels que chèvres, moutons, bison, chameaux, vaches, cochons, lapins, chevaux, rats, souris ou lamas.

Un peptide signal selon l'invention peut être utilisé soit dans la production d'une protéine possédant un peptide signal naturel, soit dans la production d'une protéine qui ne possède pas de peptide signal naturel, telle qu'une protéine de fusion ou une protéine artificielle.

Dans un mode de réalisation particulier, l'invention concerne l'utilisation d'un peptide signal tel que défini ci-dessus, dans laquelle ledit peptide signal est représenté par la formule (II) :

M(X1)iX2X3X4SX5X6X7,

dans laquelle X1, i, X2, X3 X4, X5 X6 et X7 ont les significations indiquées à la formule I.

Dans un mode de réalisation de l'utilisation d'un peptide signal tel que défini ci-dessus, le peptide X3 comprend au moins 3 leucines contigües.

Par « 3 leucines contigües », on entend trois leucines liées l'une après l'autre successivement et directement, aucun autre acide aminé n'étant inséré parmi ces trois leucines.

Dans une illustration de l'invention, le peptide signal tel que défini ci-dessus, le peptide X3 comprend au moins 3 leucines non contigües.

Par « 3 leucines non contigües », on entend qu'au moins une leucine parmi les trois n'est pas liée directement à l'une des deux autres leucines et qu'au moins un acide aminé différent de la leucine est inséré entre au moins deux leucines parmi les trois.

A titre d'exemple, trois leucines non contigües peuvent comprendre 2 leucines liées l'une à l'autre directement et successivement, une troisième leucine étant liée à ces deux leucines par l'intermédiaire d'au moins un acide aminé différent de la leucine.

A titre d'exemple, trois leucines non contigües peuvent également être séparées l'une à l'autre par au moins un acide aminé différent de la leucine. Autrement dit, ces trois leucines sont liées l'une à l'autre par au moins un acide aminé différent de la leucine situé entre chacune des leucines.

Dans un mode de réalisation particulière, l'invention concerne l'utilisation d'un peptide signal tel que défini ci-dessus, dans laquelle :
- X1, X2 et X4 ont les significations indiquées à la formule I,
- i = 1,
- X3 est un peptide constitué de leucines, représenté par Ln, dans lequel n est le nombre de leucines, n étant un nombre supérieur ou égal à 3,
- X4 est un peptide contenant de 2 à 5 acides aminés choisis parmi Ala, Thr, Ser, Gln, Ile,
- X5 est Ala,
- X6 est Gln ou Asn,
- X7 est Ala,
ledit peptide comprenant la séquence d'acides aminés suivante: X1X2LnX4SAX6A.

Dans un mode de réalisation plus particulier, l'invention concerne l'utilisation d'un peptide signal représenté par la séquence d'acides aminés SEQ ID NO: 1 (MRWSWIFLLLLSITSANA).

La demande divulgue aussi l'utilisation d'un peptide signal tel que défini ci-dessus, dans laquelle :
- X1 et X2 ont les significations indiquées à la formule I,
- i = 1,
- X3 est un peptide comprenant 3 leucines non contigües, parmi lesquelles 2 leucines sont séparées par un autre acide aminé hydrophobe choisi parmi Gly, Ala, Val, Ile, Pro, Phe, Trp,
- X4 est un peptide contenant de 2 à 5 acides aminés choisis parmi Ala, Thr, Ser, Gln, Ile,
- X5 est Val,
- X6 est His,
- X7 est Cys,
ledit peptide comprenant la séquence d'acides aminés suivante : X1X2X3X4SVHC.

La demande divulgue aussi l'utilisation d'un peptide signal représenté par la séquence d'acides aminés SEQ ID NO : 2 (MRWSWIFLFLLSITASVHC), sous réserve que ce peptide signal ne soit pas mis en oeuvre dans la production de la chaîne lourde d'un anticorps anti-AMHRII. Le peptide signal représenté par la séquence SEQ ID NO : 2 est codé par l'acide nucléique représenté par la séquence SEQ ID NO: 28 (atgcgatggagctggatctttctcttcctcctgtcaataactgcaagtgtccattgc).

La demande divulgue aussi l'utilisation d'un peptide signal tel que défini ci-dessus, dans laquelle :
- X1, X2 et X4 ont les significations identiques à la formule I,
- i = 0,
- X3 est un peptide comprenant 3 leucines non contigües, parmi lesquelles 2 leucines sont séparées par un autre acide aminé hydrophobe choisi parmi Gly, Ala, Val, Ile, Pro, Phe, Trp,
- X5 est Ala,
- X6 est Gln,
- X7 est Ala,
ledit peptide comprenant la séquence d'acides aminés suivante : X2X3X4SAQA.

La demande divulgue aussi l'utilisation d'un peptide signal représenté par la séquence d'acides aminés SEQ ID NO : 3 (MAWVWTLLFLMAAAQSAQA), sous réserve que ce peptide signal ne soit pas mis en oeuvre dans la production de la chaîne lourde d'un anticorps anti-CD5, ou d'un anticorps anti-CD23. Le peptide signal représenté par la séquence SEQ ID NO : 3 est codé par l'acide nucléique représenté par la séquence SEQ ID NO : 29 (atggcttgggtgtggaccttgctattcctgatggcagctgcccaaagtgcccaagca).

Le système d'expression *in vitro* peut être tout système d'expression connu de l'homme métier, par exemple l'expression de protéines hétérologues dans les bactéries, les levures, les cellules d'insectes ou d'autres lignées cellulaires eucaryotes.

Avantageusement, l'utilisation d'un peptide signal selon l'invention est mise en oeuvre dans une lignée cellulaire d'eucaryote supérieur.

Plus avantageusement, la susdite lignée cellulaire eucaryote supérieure peut être choisie parmi SP2/0, (SP2/0-Ag 14), NS0, autres myélomes de rat comme IR983F, des lignées humaines comme Namalwa, Wil-2, Jurkat, Molt-4, PER.C6, HEK293T/17, HEK293, HEK-293.2, Vero, Cos-1 ou Cos-7, BHK, CHO-K-1, CHO-Lec1, CHO-Lec10, CHO-Lec13, CHO Pro-5, CHO DX B11 et CHO DG44 et autres lignées comme EBx avec notamment EB66K6H6, et P3X63Ag8.653 et YB2/0, CHO-S et HEK-293F.

Un tel polypeptide recombinant d'intérêt produit à l'aide d'un peptide signal selon l'invention peut être toute protéine d'intérêt, ou faire partie d'une protéine d'intérêt. Un tel polypeptide d'intérêt peut être choisi parmi, à titre d'exemple, une hormone, une enzyme d'immunoglobuline, une immunoglobuline entière ou tout fragment dérivé d'une immunoglobuline, une protéine intervenant dans la réaction immunitaire tels que cytokines, interleukines, facteurs du complément, une protéine chimérique, ou autre protéine thérapeutique telle que des facteurs de coagulation, des protéines de la matrice extracellulaire, ou des récepteurs solubles.

Dans un mode de réalisation avantageux, un peptide signal selon l'invention permet de produire dans le milieu extracellulaire, un polypeptide recombinant d'intérêt, dans lequel les structures primaire et secondaire dudit polypeptide sont identiques à celles du polypeptide produit à l'aide de son peptide signal naturel.

Dans un mode de réalisation particulier de l'invention, le peptide signal MRWSWIFLLLLSITSANA selon l'invention permet de produire dans le milieu extracellulaire, un polypeptide recombinant d'intérêt, dans lequel les structures primaire et secondaire dudit polypeptide sont identiques à celles du polypeptide produit à l'aide de son peptide signal naturel.

La présente divulgation est également illustrée par un peptide signal comprenant une séquence d'au moins 12 acides aminés de formule III:

X1X2X3X4SX5X6X7,

dans laquelle :
- X1 est un peptide contenant de 3 à 6 acides aminés,
- X2 est un peptide contenant de 3 à 9 acides aminés hydrophobes,
- X3 est un peptide contenant de 3 à 5 acides aminés, ledit peptide comprenant au moins 3 leucines contigües,
- X4 est un peptide contenant de 2 à 5 acides aminés choisis parmi Ala, Thr, Ser, Gln, Ile,
- X5 est Ala,
- X6 est Gln ou Asn,
- X7 est Ala.

La présente invention a ainsi pour objet un peptide signal comprenant une séquence d'au moins 12 acides aminés de formule III:

X1X2X3X4SX5X6X7,

dans laquelle :
- X1 est un peptide contenant de 3 à 6 acides aminés,
- X2 est un peptide contenant de 3 à 9 acides aminés hydrophobes choisis parmi Gly, Ala, Val, Ile, Pro, Phe, Trp,
- X3 est un peptide contenant de 3 à 5 acides aminés, ledit peptide comprenant au moins 3 leucines contigües,
- X4 est un peptide contenant de 2 à 5 acides aminés choisis parmi Ala, Thr, Ser, Gln, Ile,
- X5 est Ala,
- X6 est Gln ou Asn,
- X7 est Ala.

Les leucines situées dans la région h des susdits peptides signaux permettent de créer un stretch de leucine.

Dans les peptides signaux décrits ci-dessus, la région « SAX6A » correspond au site de reconnaissance de la peptidase. L'acide aminé représenté par X6 peut être une glutamine ou une asparagine, car elles présentent des propriétés physicochimiques semblables.

Dans un mode de réalisation avantageux, les peptides signaux selon l'invention correspondent à la formule (IV) : MX1X2X3X4SX5X6X7, dans laquelle X1, X2, X3 X4, X5 X6 et X7 ont les significations indiquées à la formule III.

Dans un mode de réalisation particulier, l'invention concerne les peptides signaux correspondant à la formule III ou à la formule IV, dans lesquels:
- X3 est un peptide contenant 4 leucines contigües,
- le peptide X1 est RWS,
- X2, X4 et X6 ont les significations indiquées à la formule III,
le peptide signal répondant à la formule suivante : RWSX2X3X4SAX6A.

Dans un autre mode de réalisation particulier, l'invention concerne les peptides signaux correspondant à la formule III ou à la formule IV, dans lesquels:
- X3 est un peptide contenant 4 leucines contigües,
- le peptide X2 est WIF,
- X1, X4 et X6 ont les significations indiquées à la formule III,
le peptide signal répondant à la formule suivante : X1WIFX3X4SAX6A.

Dans un autre mode de réalisation particulier, l'invention concerne les peptides signaux correspondant à la formule III ou à la formule IV, dans lesquels:
- X3 est un peptide contenant 4 leucines contigües,
- le peptide X4 est SIT,
- X1, X2 et X6 ont les significations indiquées à la formule III,
le peptide signal répondant à la formule suivante : X1X2X3SITSAX6A.

Dans un mode de réalisation particulièrement avantageux, le peptide signal selon l'invention comprend ou consiste en la séquence d'acides aminés : MRWSWIFLLLLSITSANA (SEQ ID NO : 1).

L'invention a également pour objet les acides nucléiques codant l'un des peptides signaux répondant à la formule III ou à la formule IV.

Dans une illustration particulière de la divulgation l'acide nucléique code un peptide signal comprenant au moins 12 acides aminés de formule III: X1X2X3X4SX5X6X7, dans lequel :
- X1 est un peptide contenant de 3 à 6 acides aminés,
- X2 est un peptide contenant de 3 à 9 acides aminés hydrophobes,
- X3 est un peptide contenant de 3 à 5 acides aminés, ledit peptide comprenant au moins 3 leucines contigües,
- X4 est un peptide contenant de 2 à 5 acides aminés choisis parmi Ala, Thr, Ser, Gln, Ile,
- X5 est Ala,
- X6 est Gln ou Asn,
- X7 est Ala.

Dans un mode de réalisation particulier, l'acide nucléique selon l'invention code un peptide signal comprenant au moins 12 acides aminés de formule III: X1X2X3X4SX5X6X7, dans lequel :
- X1 est un peptide contenant de 3 à 6 acides aminés,
- X2 est un peptide contenant de 3 à 9 acides aminés hydrophobes choisis parmi Gly, Ala, Val, Ile, Pro, Phe, Trp,
- X3 est un peptide contenant de 3 à 5 acides aminés, ledit peptide comprenant au moins 3 leucines contigües,
- X4 est un peptide contenant de 2 à 5 acides aminés choisis parmi Ala, Thr, Ser, Gln, Ile,
- X5 est Ala,
- X6 est Gln ou Asn,
- X7 est Ala.

Dans un mode de réalisation plus particulier, l'acide nucléique selon l'invention code un peptide signal de formule (IV) : M X1X2X3X4SX5X6X7, dans lesquels X1, X2, X3 X4, X5 X6 et X7 ont les significations indiquées à la formule III.

Dans un mode de réalisation encore plus particulier, l'acide nucléique selon l'invention code un peptide signal répondant à la formule III ou à la formule IV, dans lequel :
- X3 est un peptide contenant 4 leucines contigües,
- le peptide X1 est RWS.

Dans un autre mode de réalisation encore plus particulier, l'acide nucléique selon l'invention code un peptide signal répondant à la formule III ou à la formule IV, dans lequel :
- X3 est un peptide contenant 4 leucines contigües,
- le peptide X2 est WIF.

Dans un autre mode de réalisation encore plus particulier, l'acide nucléique selon l'invention code un peptide signal répondant à la formule III ou à la formule IV, dans lequel :
- X3 est un peptide contenant 4 leucines contigües,
- le peptide X4 est SIT.

Les séquences nucléotidiques selon l'invention peuvent être déduites à partir des séquences d'acides aminés des peptides signaux selon l'invention. Les codons génétiques respectifs de chaque acide aminé chez les eucaryotes sont connus de l'homme de métier.

Dans un mode de réalisation particulièrement avantageux de l'invention, l'acide nucléique selon l'invention code un peptide représenté par la séquence SEQ ID NO : 1 : MRWSWIFLLLLSITSANA.

Plus particulièrement, cette séquence nucléotidique est la séquence suivante (SEQ ID NO : 4): 5'-atgcgatggagctggatcttcctgctgctgctgagcatcaccagcgccaacgcc-3'.

Les acides nucléiques codant l'un des peptides signaux selon l'invention peuvent être ajoutés à l'extrémité 5' d'un acide nucléique codant un polypeptide d'intérêt par toute méthode connue de l'homme du métier, par exemple, la technique de la PCR.

La demande divulgue aussi un précurseur d'un polypeptide recombinant d'intérêt, dans lequel le peptide signal comprend au moins 12 acides aminés de formule (I) : (X1)ᵢX2X3X4SX5X6X7, dans laquelle :
- X1 est un peptide contenant de 3 à 6 acides aminés, i égal à 0 ou 1,
- X2 est un peptide contenant de 3 à 9 acides aminés hydrophobes,
- X3 est un peptide contenant de 3 à 5 acides aminés, ledit peptide comprenant au moins 3 leucines, contigües ou non,
- X4 est un peptide contenant de 2 à 5 acides aminés choisis parmi Ala, Thr, Ser, Gln, Ile, Met,
- X5 est Ala ou Val,
- X6 est Gln, Asn ou His,
- X7 est Ala ou Cys,
sous réserve que lorsque ledit peptide signal est issu d'un précurseur naturel d'une protéine déterminée, ledit polypeptide d'intérêt est différent de ladite protéine, et lorsque le peptide signal est MAWVWTLLFLMAAAQSAQA, ledit polypeptide d'intérêt n'est pas un anticorps anti-CD23, n'importe quel polypeptide d'intérêt pouvant être lié à l'un desdits peptides signaux pour être sécrété dans le milieu extracellulaire.

La présente invention a aussi pour objet un précurseur d'un polypeptide recombinant d'intérêt, dans lequel un peptide signal comprend au moins 12 acides aminés de formule (I) : (X1)iX2X3X4SX5X6X7, dans laquelle :
- X1 est un peptide contenant de 3 à 6 acides aminés, i égal à 0 ou 1,
- X2 est un peptide contenant de 3 à 9 acides aminés hydrophobes choisis parmi Gly, Ala, Val, Ile, Pro, Phe, Trp,
- X3 est un peptide contenant de 3 à 5 acides aminés, ledit peptide comprenant au moins 3 leucines contigües,
- X4 est un peptide contenant de 2 à 5 acides aminés choisis parmi Ala, Thr, Ser, Gln, Ile, Met,
- X5 est Ala ou Val,
- X6 est Gln, Asn ou His,
- X7 est Ala ou Cys,
sous réserve que lorsque ledit peptide signal est issu d'un précurseur naturel d'une protéine déterminée, ledit polypeptide d'intérêt est différent de ladite protéine, n'importe quel polypeptide d'intérêt pouvant être lié à l'un desdits peptides signaux pour être sécrété dans le milieu extracellulaire, et notamment dans lequel le peptide signal est l'un des peptides signaux de l'invention tels que définis précédemment.

Dans un mode de réalisation particulier, un précurseur selon l'invention comprend un peptide signal répondant à la formule III ou à la formule IV décrites ci-dessus, dans lequel X1, X2, X3, X4, X5, X6 et X7 ont les significations indiquées à la formule III.

Dans une illustration de la description, un précurseur comprend un peptide signal comprenant la séquence d'acides aminés suivante : X1X2LnX4SAX6A, dans laquelle :
- X1 est un peptide contenant de 3 à 6 acides aminés,
- X2 est un peptide contenant de 3 à 9 acides aminés hydrophobes,
- n est le nombre de leucines, n étant un nombre supérieur ou égal à 3,
- X4 est un peptide contenant de 2 à 5 acides aminés choisis parmi Ala, Thr, Ser, Gln, Ile,
- X6 est Gln ou Asn.
   Dans un mode de réalisation particulier, un précurseur selon **l'invention** comprend un peptide signal comprenant la séquence d'acides aminés suivante : X1X2LnX4SAX6A, dans laquelle :
- X1 est un peptide contenant de 3 à 6 acides aminés,
- X2 est un peptide contenant de 3 à 9 acides aminés hydrophobes choisis parmi Gly, Ala, Val, Ile, Pro, Phe, Trp,
- n est le nombre de leucines, n étant un nombre supérieur ou égal à 3,
- X4 est un peptide contenant de 2 à 5 acides aminés choisis parmi Ala, Thr, Ser, Gln, Ile,
- X6 est Gln ou Asn.

Dans un mode de réalisation particulièrement avantageux, un précurseur d'un polypeptide d'intérêt selon l'invention comprend un peptide signal consistant en la séquence d'acides aminés suivante : MRWSWIFLLLLSITSANA (SEQ ID NO : 1).

Dans une illustration décrite un précurseur comprend un peptide signal comprenant la séquence d'acides aminés suivante : X1X2X3X4SVHC, dans laquelle :
- X1 est un peptide contenant de 3 à 6 acides aminés,
- X2 est un peptide contenant de 3 à 9 acides aminés hydrophobes,
- X3 est un peptide comprenant 3 leucines non contigües, parmi lesquelles 2 leucines sont séparées par un autre acide aminé hydrophobe choisi parmi Gly, Ala, Val, Ile, Pro, Phe, Trp,
- X4 est un peptide contenant de 2 à 5 acides aminés choisis parmi Ala, Thr, Ser, Gln,
sous réserve que lorsque ledit peptide signal est issu d'un précurseur naturel d'une protéine déterminée, ledit polypeptide d'intérêt est différent de ladite protéine.

Dans une illustration décrite un précurseur d'un polypeptide d'intérêt comprend un peptide signal consistant en la séquence d'acides aminés suivante : MRWSWIFLFLLSITASVHC (SEQ ID NO : 2), sous réserve que ledit polypeptide d'intérêt soit différent de la chaîne gamma d'un anticorps anti-AMHRII.

Dans une illustration décrite un précurseur comprend un peptide signal comprenant la séquence d'acides aminés suivante : X2X3X4SAQA, dans laquelle :
- X2 est un peptide contenant de 3 à 9 acides aminés hydrophobes,
- X3 est un peptide comprenant 3 leucines non contigües, parmi lesquelles 2 leucines sont séparées par un autre acide aminé hydrophobe choisi parmi Gly, Ala, Val, Ile, Pro, Phe, Trp, Leu,
- X4 est un peptide contenant de 2 à 5 acides aminés choisis parmi Ala, Thr, Ser, Gln, Met,
sous réserve que lorsque ledit peptide signal est issu d'un précurseur naturel d'une protéine déterminée, ledit polypeptide d'intérêt est différent de ladite protéine.

Dans un mode de réalisation particulièrement avantageux, un précurseur d'un polypeptide d'intérêt selon l'invention comprend un peptide signal consistant en la séquence d'acides aminés suivante : MAWVWTLLFLMAAAQSAQA (SEQ ID NO : 3), sous réserve que ledit polypeptide d'intérêt soit différent de la chaîne gamma d'un anticorps anti-CD5, et que ledit polypeptide d'intérêt soit différent d'un anticorps anti-CD23.

Dans un mode de réalisation particulier, un tel précurseur selon l'invention est un précurseur d'un polypeptide recombinant d'intérêt choisi parmi une hormone, une enzyme, une chaîne d'immunoglobuline, une immunoglobuline entière ou tout fragment dérivé d'une immunoglobuline, une protéine intervenant dans la réaction immunitaire telle que des cytokines, interleukines, facteurs du complément, une protéine chimérique, ou d'autres protéines thérapeutiques telle que des facteurs de coagulation, protéines de la matrice extracellulaire ou récepteurs solubles.

Dans un mode de réalisation plus particulier, un tel précurseur selon l'invention est un précurseur d'un polypeptide recombinant d'intérêt choisi parmi une chaîne légère d'un anticorps, et/ou une chaîne lourde d'un anticorps.

La présente invention a également pour objet un acide nucléique codant l'un des précurseurs d'un polypeptide recombinant d'intérêt selon la présente invention.

La présente invention a aussi pour objet un vecteur d'expression comprenant un acide nucléique tel que décrit ci-dessus, codant un précurseur d'un polypeptide recombinant d'intérêt tel que décrit ci-dessus.

Dans un mode de réalisation particulier, le vecteur selon l'invention comprend en outre les moyens génétiques, en particulier les origines de réplication, les promoteurs, permettant de contrôler l'expression du susdit polypeptide recombinant d'intérêt.

Ledit vecteur d'expression selon l'invention peut être capable d'exprimer le susdit polypeptide recombinant d'intérêt dans une lignée cellulaire d'eucaryote supérieur après une transfection transitoire ou stable, ladite lignée pouvant être avantageusement choisie parmi SP2/0, (SP2/0-Ag 14), NS0, autres myélomes de rat comme IR983F, des lignées humaines comme Namalwa, Wil-2, Jurkat, Molt-4, PER.C6, HEK293T/17, HEK293, HEK-293.2, Vero, Cos-1 ou Cos-7, BHK, CHO-K-1, CHO-Lec1, CHO-Lec10, CHO-Lec13, CHO Pro-5, CHO DX B11 et CHO DG44 et autres lignées comme EBx avec notamment EB66K6H6, et P3X63Ag8.653, YB2/0, CHO-S et HEK-293F.

La présente divulgation est aussi illustrée par un procédé de production d'un polypeptide recombinant d'intérêt, comprenant :
- l'ajout d'un acide nucléique codant un peptide signal comprenant au moins 12 acides aminés de formule (I) :

   (X1)ᵢX2X3X4SX5X6X7,

   dans laquelle :
   - X1 est un peptide contenant de 3 à 6 acides aminés, i égal à 0 ou 1,
   - X2 est un peptide contenant de 3 à 9 acides aminés hydrophobes,
   - X3 est un peptide contenant de 3 à 5 acides aminés, ledit peptide comprenant au moins 3 leucines, contigües ou non,
   - X4 est un peptide contenant de 2 à 5 acides aminés choisis parmi Ala, Thr, Ser, Gln, Ile, Met,
   - X5 est Ala ou Val,
   - X6 est Gln, Asn ou His,
   - X7 est Ala ou Cys,
   à l'extrémité 5' d'un acide nucléique codant un polypeptide recombinant d'intérêt, pour obtenir un acide nucléique codant le précurseur du susdit polypeptide recombinant d'intérêt, sous réserve que lorsque ledit peptide signal est issu d'un précurseur naturel d'une protéine déterminée, ledit polypeptide d'intérêt est différent de ladite protéine,
- le clonage de l'acide nucléique artificiel obtenu dans l'étape précédente à un vecteur d'expression, pour obtenir un vecteur capable d'exprimer le précurseur du susdit polypeptide recombinant d'intérêt, et
- la transfection d'une lignée cellulaire d'eucaryote supérieur par le vecteur d'expression comprenant ladite séquence nucléotidique artificielle et l'expression de ladite séquence nucléotidique,
- la récupération du polypeptide recombinant d'intérêt secrété dans le milieu de culture.

La présente invention concerne aussi un procédé de production d'un polypeptide recombinant d'intérêt, comprenant :
- l'ajout d'un acide nucléique codant un peptide signal comprenant au moins 12 acides aminés de formule (I) :

   (X1)ᵢX2X3X4SX5X6X7,

   dans laquelle :
   - X1 est un peptide contenant de 3 à 6 acides aminés, i égal à 0 ou 1,
   - X2 est un peptide contenant de 3 à 9 acides aminés hydrophobes choisis parmi Gly, Ala, Val, Ile, Pro, Phe, Trp,
   - X3 est un peptide contenant de 3 à 5 acides aminés, ledit peptide comprenant au moins 3 leucines, contigües,
   - X4 est un peptide contenant de 2 à 5 acides aminés choisis parmi Ala, Thr, Ser, Gln, Ile, Met,
   - X5 est Ala ou Val,
   - X6 est Gln, Asn ou His,
   - X7 est Ala ou Cys,
   à l'extrémité 5' d'un acide nucléique codant un polypeptide recombinant d'intérêt, pour obtenir un acide nucléique codant le précurseur du susdit polypeptide recombinant d'intérêt, sous réserve que lorsque ledit peptide signal est issu d'un précurseur naturel d'une protéine déterminée, ledit polypeptide d'intérêt est différent de ladite protéine,
- le clonage de l'acide nucléique artificiel obtenu dans l'étape précédente à un vecteur d'expression, pour obtenir un vecteur capable d'exprimer le précurseur du susdit polypeptide recombinant d'intérêt, et
- la transfection d'une lignée cellulaire d'eucaryote supérieur par le vecteur d'expression comprenant ladite séquence nucléotidique artificielle et l'expression de ladite séquence nucléotidique,
- la récupération du polypeptide recombinant d'intérêt secrété dans le milieu de culture.

La récupération d'un polypeptide recombinant peut être effectuée par toute méthode connue de l'homme du métier, par exemple la précipitation, la centrifugation, l'élution par chromatographie. Cette étape peut être également associée à une étape de purification du polypeptide.

Un vecteur d'expression mis en oeuvre dans le susdit procédé selon l'invention peut être tout vecteur d'expression connu de l'homme de métier, par exemple un vecteur permettant d'exprimer *in vitro* un polypeptide dans une lignée cellulaire d'eucaryote supérieur en transfection transitoire ou stable.

Dans un mode de réalisation particulier du procédé de production selon l'invention, la lignée cellulaire eucaryote est choisie parmi les lignées cellulaires : SP2/0, (SP2/0-Ag 14), NS0, autres myélomes de rat comme IR983F, des lignées humaines comme Namalwa, Wil-2, Jurkat, Molt-4, PER.C6, HEK293T/17, HEK293, HEK-293.2, Vero, Cos-1 ou Cos-7, BHK, CHO-K-1, CHO-Lec1, CHO-Lec10, CHO-Lec13, CHO Pro-5, CHO DX B11 et CHO DG44 et autres lignées comme EBx avec notamment EB66K6H6, et P3X63Ag8.653, YB2/0, CHO-S et HEK-293F.

Dans un mode de réalisation particulier du procédé de production selon l'invention, le polypeptide recombinant d'intérêt est choisi parmi une hormone, une enzyme, une chaîne d'immunoglobuline, une immunoglobuline entière ou tout fragment dérivé d'une immunoglobuline, une protéine intervenant dans la réaction immunitaire, tels que cytokines, interleukines, facteurs du complément, une protéine chimérique, ou toute autres protéines thérapeutiques, telle que des facteurs de coagulation, protéines de la matrice extracellulaire, récepteurs solubles.

Dans un mode de réalisation particulier de l'invention, la présente invention concerne un procédé de production d'anticorps recombinants :
- l'ajout d'un acide nucléique codant le peptide signal MRWSWIFLLLLSITSANA à l'extrémité 5' d'un acide nucléique codant une chaine d'immunoglobuline, pour obtenir un acide nucléique codant le précurseur d'une chaine d'immunoglobuline,
- le clonage de l'acide nucléique artificiel obtenu dans l'étape précédente à un vecteur d'expression, pour obtenir un vecteur capable d'exprimer le précurseur d'une chaine d'immunoglobuline,
- la transfection d'une lignée cellulaire d'eucaryote supérieur choisi parmi PER.C6, YB2/0, CHO-S et HEK 293, par le ou les vecteurs d'expressions comprenant ladite séquence nucléotidique artificielle et l'expression de ladite séquence nucléotidique,
- la récupération de l'anticorps secrété dans le milieu de culture.

Dans un mode de réalisation plus particulier de l'invention et dans le cadre de la production d'un anticorps, le peptide signal utilisé pour la production de la chaîne lourde est le même que celui utilisé pour la production de la chaîne légère.

Dans un mode de réalisation encore plus particulier de l'invention pour la production d'un anticorps, le peptide signal MRWSWIFLLLLSITSANA est utilisé pour produire à la fois la chaîne lourde et la chaîne légère dudit anticorps d'intérêt.

La présente invention est illustrée, uniquement à titre d'exemple, par les figures et les exemples ci-dessous.

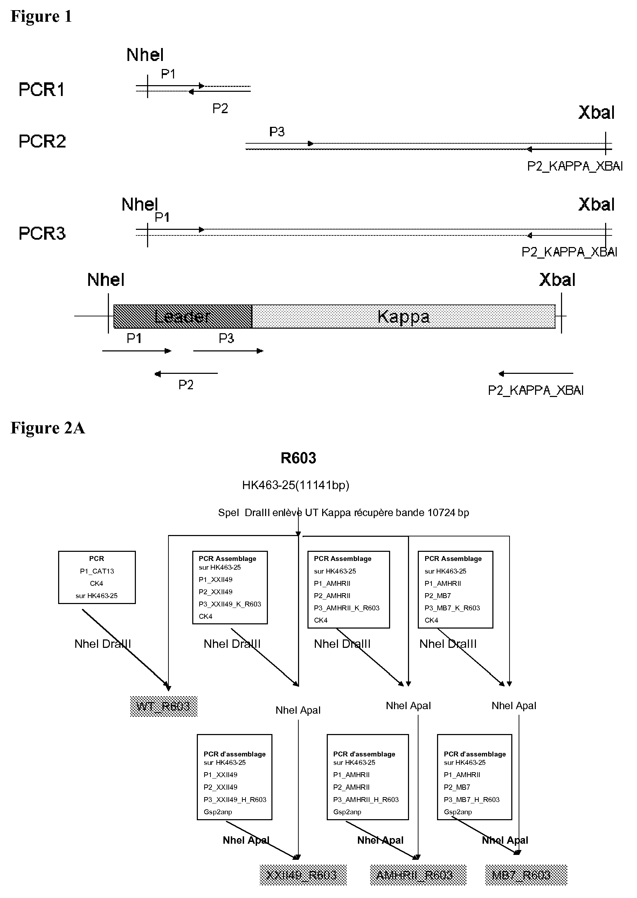

**Figure 1** **:** la figure 1 représente le principe utilisé pour l'ajout des peptides signaux en 5' de la chaîne kappa T125. Les flèches indiquent les différentes amorces utilisées pour la PCR d'assemblage.
**Figure 2A** **:** la figure 2A représente le principe utilisé pour préparer les vecteurs d'expression codant l'anticorps anti-CD20 (R603) et permettant d'évaluer le pouvoir sécréteur des PS sur IgG entières. Les encadrés représentent les parties variables chaines lourde ou légère à cloner dans le vecteur. Les vecteurs finaux qui font l'objet des évaluations sont surlignés en gris.

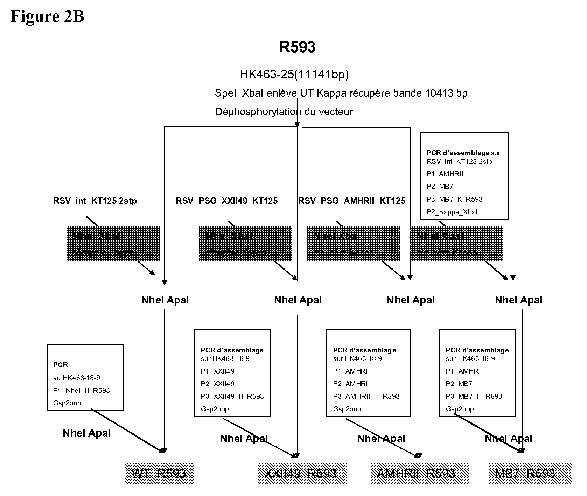

**Figure 2B** **:** la figure 2A représente le principe utilisé pour préparer les vecteurs d'expression codant l'anticorps anti-RhD (R593) et permettant d'évaluer le pouvoir sécréteur des PS sur IgG entières. Les encadrés représentent les parties variables chaines lourde ou légère à cloner dans le vecteur. Les vecteurs finaux qui font l'objet des évaluations sont surlignés en gris.

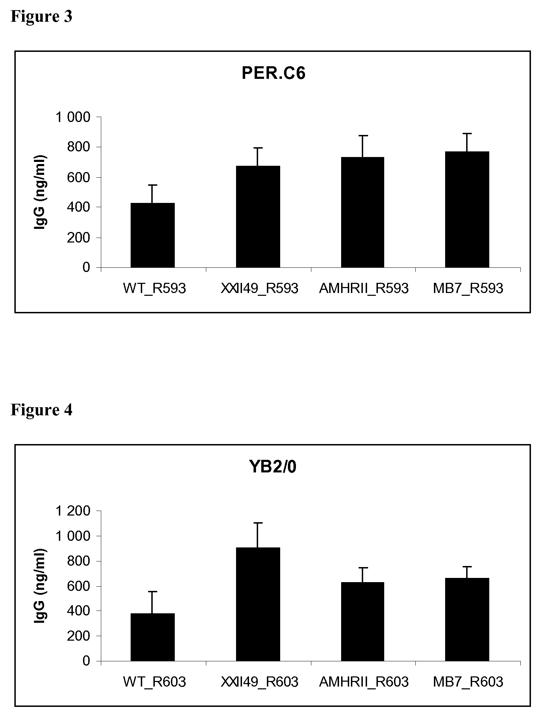

**Figure 3** **:** la figure 3 présente une comparaison de l'effet de 3 peptides signaux, dont 12G4 (représenté par la séquence SEQ ID NO : 2), XXII49 (représenté par la séquence SEQ ID NO : 3) et MB7 (représenté par la séquence SEQ ID NO : 1), sur la sécrétion d'un anticorps anti-RhD entier (R593), dans la lignée cellulaire PER.C6 évaluée en transfection transitoire. Les quatre colonnes, de gauche à droite, représentent respectivement le taux de sécrétion de l'anticorps R593 lié respectivement à ses peptides signaux initiaux (chaine lourde, chaine légère), au peptide signal 12G4, au peptide signal XXII49 et au peptide signal selon l'invention MB7. L'axe des ordonnées représente la concentration en anticorps R593 secrété dans le milieu de culture.
**Figure 4** **:** la figure 4 présente une comparaison de l'effet de 3 peptides signaux, dont 12G4, XXII49 et MB7, sur la sécrétion d'un anticorps anti-CD20 entier (R603), dans la lignée cellulaire YB2/0 évaluée en transfection transitoire. Les quatre colonnes, de gauche à droite, représentent respectivement le taux de sécrétion de l'anticorps R603 lié respectivement à ses peptides signaux initiaux (chaine lourde, chaine légère), au peptide signal 12G4, au peptide signal XXII49 et au peptide signal selon l'invention MB7. L'axe des ordonnées représente la concentration en anticorps R603 secrété dans le milieu de culture.

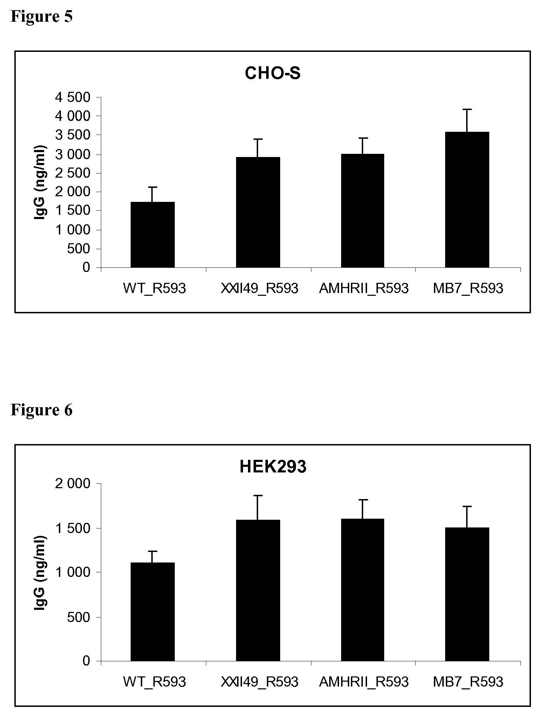

**Figure 5** **:** la figure 5 présente une comparaison de l'effet de 3 peptides signaux, dont 12G4, XXII49 et MB7, sur la sécrétion d'un anticorps anti-RhD(R593) entier, dans la lignée cellulaire CHO-S évaluée en transfection transitoire. Les quatre colonnes, de gauche à droite, représentent respectivement le taux de sécrétion de l'anticorps R593 lié respectivement à ses peptides signaux initiaux (chaine lourde, chaine légère), au peptide signal 12G4, au peptide signal XXII49 et au peptide signal selon l'invention MB7. L'axe des ordonnées représente la concentration en anticorps R593 secrété dans le milieu de culture.
**Figure 6** **:** la figure 6 présente une comparaison de l'effet de 3 peptides signaux, dont 12G4, XXII49 et MB7, sur la sécrétion d'un anticorps anti-RhD (R593) entier, dans la lignée cellulaire HEK293 évaluée en transfection transitoire. Les quatre colonnes, de gauche à droite, représentent respectivement le taux de sécrétion de l'anticorps R593 lié respectivement à ses peptides signaux initiaux (chaine lourde, chaine légère), au peptide signal 12G4, au peptide signal XXII49 et au peptide signal selon l'invention MB7. L'axe des ordonnées représente la concentration en anticorps R593 secrété dans le milieu de culture.

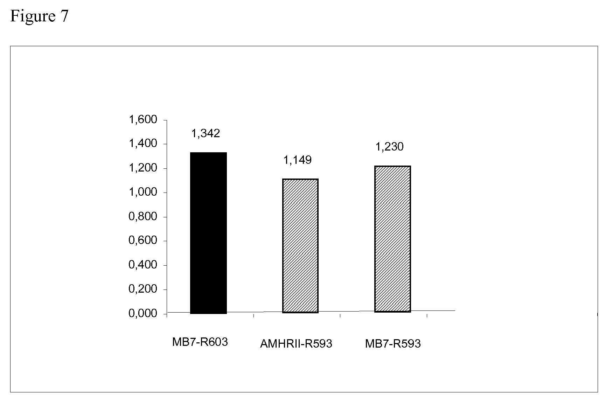

**Figure 7** **:** la figure 7 présente la comparaison des productions moyennes en fonction des différents peptides signaux par rapport aux peptides signaux naturels dans la lignée cellulaire PER.C6. L'axe des ordonnées représente le rapport entre le taux de sécrétion de l'anticorps lié à un peptide signal selon l'invention (12G4 ou MB7) et le taux de sécrétion de l'anticorps lié à ses peptides signaux naturels. Les trois colonnes, de gauche à droite, représentent respectivement le taux de sécrétion de l'anticorps R603 lié au peptide signal MB7, et le taux de sécrétion de l'anticorps R593 lié respectivement au peptide signal 12G4, et au peptide signal MB7.

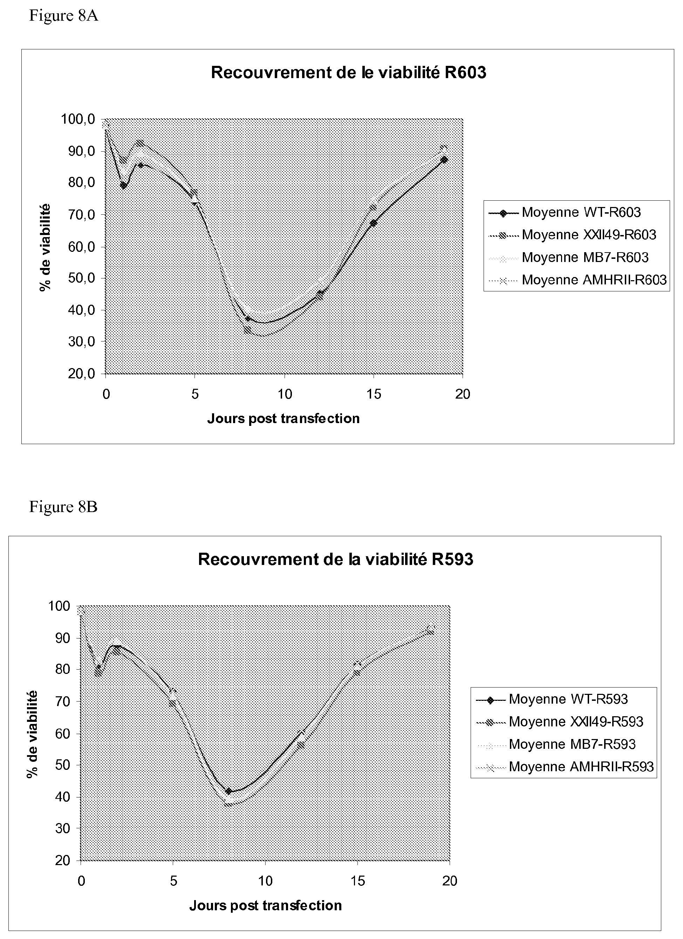

**Figure 8A** **:** la figure 8A montre le recouvrement de la viabilité après transfection pour l'anti-CD20 lié respectivement à son peptide signal naturel (losange), au peptide signal XXII49 (carré), au peptide signal MB7 (triangle), et au peptide signal 12G4 (croix). L'axe des ordonnées représente le pourcentage de viabilité. L'axe des abscisses représente les jours post transfection. Les résultats sont la moyenne sur les trois Pools stables.
**Figure 8B** **:** la figure 8A montre le recouvrement de la viabilité après transfection pour l'anti-Rh(D) lié respectivement à son peptide signal naturel (losange), au peptide signal XXII49 (carré), au peptide signal MB7 (triangle), et au peptide signal 12G4 (croix). L'axe des ordonnées représente le pourcentage de viabilité. L'axe des abscisses représente les jours post transfection. Les résultats sont la moyenne sur les trois Pools stables.

### Les exemples

### Exemple 1 : Matériels et Méthodes

### 1.1 Peptides signaux analysés

Sept peptides signaux issus respectivement d'une chaîne polypeptidique portant naturellement un peptide signal sont choisis par les Inventeurs pour analyser le pouvoir sécréteur de ces 7 différents peptides signaux.

Ces 7 chaînes polypeptidiques sont respectivement la chaîne gamma d'un anticorps anti-AMHRII (12G4, I-3673 CNCM) la chaîne gamme d'un anticorps anti-CD5 (XXII49, donnée non publiée), la chaîne gamma d'un anticorps anti-RhD (D29, FR 2861078), la chaîne gamme d'un anti-récepteur LDL (5E5, I-3488 CNCM), la chaîne kappa d'un anticorps anti-CD20 (Cat13, fournisseur: DSMZ, ref. ACC 474), la chaîne α du récepteur TCR de lymphocyte T² (HAVT20, numéro d'accession Genbank H32536) et l'érythropoiétine humaine (EPO, numéro d'accession Genbank AAI43226).

Le pouvoir sécréteur d'un peptide signal est apprécié selon deux aspects, à savoir la capacité d'adressage et la capacité à être clivé.

Des descriptions et prédictions du pouvoir sécréteur d'un peptide signal sont analysées préalablement (Emanuelsson et al., Nature Protocols 2, 953-971 (2007)).

Les peptides signaux (PS) ont été soumis dans l'algorithme SignalP V3.0 sous forme de protéine de fusion PS/chaine légère de l'anticorps anti-Rh(D) T125 (FR2807767).

### 1.2 Construction des vecteurs d'expression pour l'évaluation des PS sur l'expression de la chaîne légère de l'anticorps anti-Rh(D) T125

La méthode est identique pour chaque PS, elle se décompose en deux séries de PCR. Une première PCR est effectuée avec les amorces P1 et P2, qui permettent la synthèse des 40 à 50 bases du PS et l'intégration du site NheI en 5'de la séquence. Une deuxième PCR est réalisée en parallèle avec les amorces P3 et P2_Kappa_XbaI et pour matrice le plasmide RSV-int_KT125 2stp. Elle permettra par la suite la jonction entre le PS et la chaîne kappa et d'intégrer en 3' le site de clonage XbaI. Une troisième PCR est ensuite effectuée. Cette PCR d'assemblage est réalisée avec les amorces P1 et P2_Kappa_XbaI (Figure 1).

Afin de limiter les erreurs d'incorporation de nucléotides, l'enzyme Proofreading (proofstart Taq, Qiagen) est utilisé pour l'amplification d'ADN, et le nombre de cycles est limité.

Les PCR sont réalisées dans les conditions suivantes :
Activation enzyme 94°C, 5min 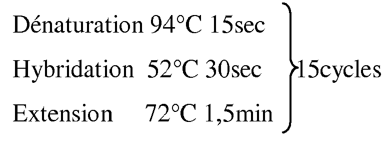
Extension finale 72°C 2min

**Les amorces utilisées sont ci-dessous :**
*** Pour la construction de la chaîne de fusion PS(12G4)/kappa(T125)**
   P1_12G4 (SEQ ID NO : 5) 5'-CTCTTGCTAGCGCCGCCACCATGCGATGGAGCTGGATCTT-3'
   P2_12G4 (SEQ ID NO : 6) 5'-CACTTGCAGTTATTGACAGGAGGAAGAGAAAGATCCAGCT-3'
   P3_12G4 (SEQ ID NO : 7) 5'-ACTGCAAGTGTCCATTGCGCCATCCGGATGACCCAGTCTC-3'
*** Pour la construction de la chaîne de fusion PS(XXII49)/kappa(T125)**
   P1_XXII49 (SEQ ID NO : 8) 5'-CTCTTGCTAGCGCCGCCACCATGGCTTGGGTGTGGACCTT-3'
   P2_XXII49 (SEQ ID NO : 9) 5'-CACTTTGGGCAGCTGCCATCAGGAATAGCAAGGTCCACAC-3'
   P3_XXII49 (SEQ ID NO : 10) 5'-GCCCAAAGTGCCCAAGCAGCCATCCGGATGACCCAGTCTC-3'
*** Pour la construction de la chaîne de fusion PS(D29)/kappa(T125)**
   P1_D29 (SEQ ID NO : 11) 5'-CTCTTGCTAGCGCCGCCACCATGGAGCTTGGGCTGAGCTG-3'
   P2_D29 (SEQ ID NO : 12) 5'-ACACCTCTTAAAAGAGCAACGAGAAAAACCCAGCTCAGCCC-3'
   P3_D29 (SEQ ID NO : 13) 5'-TTAAGAGGTGTCCAGTGTGCCATCCGGATGACCCAGTCTC-3'
*** Pour la construction de la chaîne de fusion PS(5E5)/kappa(T125)**
   P1_5E5 (SEQ ID NO : 14) 5'-CTCTTGCTAGCGCCGCCACCATGGGTTGGAGCTGTATCAT-3'
   P2_5E5 (SEQ ID NO : 15) 5'-ACACCTGTAGCTGTTGCTACCAGAAAGAAGATGATACAGCTC-3'
   P3_5E5 (SEQ ID NO : 16) 5'-AGCTACAGGTGTGCACTCCGCCATCCGGATGACCCAGTCTC-3'
*** Pour la construction de la chaîne de fusion PS(CAT13)/kappa(T125)**
   P1_CAT13 (SEQ ID NO : 17) : 5'-CTCTTGCTAGCGCCGCCACCATGGATTTTCAAGTGCAGATTTTC-3'
   P2_CAT13 (SEQ ID NO : 18): 5'-CATTATGACTGAAGCACTGATTAGCAGGAAGCTGAAAATCTGCAC-3'
   P3_CAT13 (SEQ ID NO : 19): 5'-CTTCAGTCATAATGTCCAGAGGAGCCATCCGGATGACCCAGTCTC-3'
*** Pour la construction de la chaîne de fusion PS(EPO)/kappa(T125)**
   P1_EPO(H) (SEQ ID NO : 20) : 5'-CTCTTGCTAGCGCCGCCACCATGGGGGTGCACGAATGTCCTGCCTGGCTG-3'
   P2_EPO(H) (SEQ ID NO : 21) : 5'-CAGAGGGAGCGACAGCAGGGACAGGAGAAGCCACAGCCAGGCAGGA-3'
   P3_EPO(H) (SEQ ID NO : 22) : 5'-TCGCTCCCTCTGGGCCTCCCAGTCCTGGGCGCCATCCGGATGACCCAGTCTC-3'
*** Pour la construction de la chaîne de fusion PS(HAVT20)/kappa(T125)**
   P1_HAVT20 (SEQ ID NO : 23) : 5'-CTCTTGCTAGCGCCGCCACCATGGCATGCCCTGGCTTCCTGT-3'
   P2_HAVT20 (SEQ ID NO : 24) : 5'-AATTCAAGACAGGTGGAGATCACAAGTGCCCACAGGAAGCCAG-3'
   P3_HAVT20 (SEQ ID NO : 25) : 5'-CCTGTCTTGAATTTTCCATGGCTGCCATCCGGATGACCCAGTCTC-3'
   P2_KAPPA_Xba1(SEQ ID NO : 26) : 5'-GCGAGCTCTAGAGTTCACTAACACTCTCCCCTGTTGAAGCTC-3'

Le vecteur RSV-int_KT125 2stp est digéré par NheI et XbaI. Un fragment de 725 bases, correspondant à la séquence nucléotidique codant la chaîne légère de T125, est éliminé. Un fragment de 5028 bases est récupéré. Le produit de PCR ainsi préparé est également digéré par NheI et XbaI. Les fragments après digestion sont récupérés et purifiés par Nuleospin® Extract (Clonetech). Le produit de PCR ainsi digéré est cloné entre les sites NheI et XbaI dans le vecteur d'expression RSV-int KT125-2stp en lieu et place de la chaine légère déjà présente. L'insertion correcte du produit PCR est vérifiée par une PCR à l'aide de deux amorces : P1 et P2_KAPPA_Xba1. Un amplicon d'environ 750 à 760 bases suivant les peptides signaux doit être obtenu, lorsque le vecteur est construit correctement. L'insertion correcte du produit PCR dans le vecteur est également vérifiée par le séquençage utilisant l'amorce 2BGHPA représentée par la séquence SEQ ID NO: 27 (5'-CAGATGGCTGGCAACTAGAA-3').

### 1.3 Construction des vecteurs d'expression pour l'évaluation des PS sur l'expression des anticorps anti-Rh(D) T125 et anti-CD20

Sur le même principe que précédemment (section 1.2), les PS de fanti-AMHRII, de l'anti-CD5, et du PS artificiel MB7 sont ajoutés aux chaines lourde et légère des anticorps anti-Rh(D) T125 et anti-CD20 par PCR d'assemblage (Tableau 3), PCR conventionnelle (Tableau 2) ou simple clonage (Tableau1) des fragments générés dans la première partie d'étude selon les tableaux ci- dessous:

**Tableau 1**

| Chaines | | | | | Vecteur final |
|---|---|---|---|---|---|
| Nom | Vecteur | Enzyme1 | Enzyme2 | Taille (pb) | nom |
| K_WT_R593 | RSV_int_KT125_2STP | Nhel | Xbal | 725 | WT_R593 |
| K_XXII49_R593 | RSV_PSG_XXII49_KT125 | Nhel | Xbal | 722 | XXII49_R593 |
| K_AMHRII_R593 | RSV_PSG_AMHRII_KT125 | Nhel | Xbal | 722 | AMHRII_R593 |

**Tableau 2**

| Chaines | | | | | Vecteur final |
|---|---|---|---|---|---|
| Nom | Vecteur | Primer1 | Primer2 | Taille (pb) | nom |
| H_WT_R593 | H K463-18-9 | P1_NheI_H_R593 | GSP2ANP | 555 | WT_R593 |
| K_WT_R603 | HK463-25 | P1_CAT13 | CK4 | 509 | WT_R603 |

**Tableau 3**

| | | PCR1 | | | PCR2 | | | | |
|---|---|---|---|---|---|---|---|---|---|
| | | PCR3 d'assemblage | | | | | | | |
| vecteur | chaîne | amorce 5' | amorce 3' | taille ampliconl (pb) | amorce 5' | amorce 3' | taille amplicon2 (pb) | taille finale (pb) | taille après digestion (pb) |
| XXII49_R603 | chaîne lourde | P1_XXII49 | P2_XXII49 | 69 | P3_XXII49 _H_R603 | GSP2ANP | 472 | 531 | 441 |
| | chaîne légère | | | | P3_XXII49 _K_R603 | CK4 | 441 | 500 | 408 |
| AMHRII_R603 | chaîne lourde | P1-AMHRII | P2-AMHRII | 69 | P3_AMHRII _H_R603 | GSP2ANP | 472 | 531 | 441 |
| | chaîne légère | | | | P3_AMHRII _K_R603 | CK4 | 441 | 500 | 408 |
| MB7_R603 | chaîne lourde | P1-AMHRII | P2_MB7 | 63 | P3_MB7 _H_R603 | GSP2ANP | 475 | 528 | 438 |
| | chaîne légère | | | | P3_MB7 _K_R603 | CK4 | 444 | 497 | 405 |
| XXII49_R593 | chaîne lourde | P1_XXII49 | P2_XXII49 | 69 | P3_XXII49 _H_R593 | GSP2ANP | 496 | 555 | 465 |
| AMHRII_R593 | chaîne lourde | P1-AMHRII | P2-AMHRII | 69 | P3_AMHRII _H_R593 | GSP2ANP | 496 | 555 | 465 |
| MB7_R593 | chaîne lourde | P1-AMHRII | P2_MB7 | 63 | P3_MB7 _H_R593 | GSP2ANP | 499 | 552 | 462 |
| | chaîne légère | | | | P3_MB7 _K_R593 | P2_Kappa_Xbal | 683 | 736 | 719 |

Chaque paire de chaine lourde et légère fusionnée avec un PS à évaluer est ensuite clonée dans le vecteur HK463-25 de façon séquentielle selon le diagramme de la figure 2A ou de la figure 2B. La partie variable de chaque chaine légère est amplifiée selon les tableaux ci-dessus puis est digérée par NheI/DraIII avant d'être ligué dans le vecteur, lui-même digéré par SpeI/DraIII. Un screening par PCR et digestion enzymatique est ensuite réalisé pour identifier les clones bactériens résultant de la transformation avec le vecteur. Les clones correctement générés sont ensuite manipulés pour y insérer la partie variable de la chaine lourde. Ceci se fait par digestion NheI/ApaI du vecteur et du produit de PCR avant une ligation, transformation bactérienne et screening pour identifier les clones bactériens conformes.

Les différents vecteurs ainsi construits ont été ensuite évalués en transfection transitoire dans YB2/0, PER.C6, CHO-S et HEK ou en transfection stable dans les lignées YB2/0 et PER.C6.

### 1.4 Transfection transitoire

Dans YB2/0, les cellules parentales sont ensemencées la veille de la transfection (J-1) à 2^{E}5 cv/ml en EMS (Invitrogen, milieu à façon) + 5% SVF (Invitrogen) en Flask. Le jour de l'électroporation (J0), centrifugation de 4^{E}6 cellules par cuvette (Biorad) de 4 mm repris dans 100µl de tampon V (Cell line nucleofector kitV, Lonza) qui sont nucléofectées par AMAXA avec 4 µg d'ADN plasmidique en utilisant le programme T020 de l'appareil. Les cellules sont cultivées en puits plaque P6 à 37°C, 7% de CO₂ dans 3 ml de milieu EMS + 5% de SVF. Les surnageants sont récoltés pour dosage ELISA à J+5.

Dans PER.C6 adhérente, les cellules parentales sont ensemencées 24H avant la transfection (J-1) en plaques de 24 puits à 1^{E}6 cv/ml dans DMEM (Fisher Bioblck Scientific) + 10% SVF. Le jour de la transfection un complexe FuGENE®HD (Roche)/ADN, au ratio 6:2, est formé en DMEM pendant 15 min à température ambiante. Le complexe (25µl) est ensuite déposé sur les cellules en présence de 250µl de DMEM + 10% SVF frais et incubé pendant 4h à 37°C et 10% de CO₂. A l'issue de cette période, 250µl de milieu complet sont ajoutés et les surnageants sont récoltés à J+5 pour évaluation de molécule sécrété dans le milieu.

Dans CHO-S, les PS sont évalués en transfection transitoire selon le protocole du kit FreeStyle (Invitrogen). Les cellules parentales sont ensemencées 24H avant la transfection (J-1) en Erlenmeyer (VWR) à 6^{E}5 cell/ml en FreeStyle CHO EM (Fisher Bioblock scientific) et incubées à 120 tr/min 37°C, 8% CO₂. Le jour de la transfection un complexe FreeStyle MAX Reagent (Fisher Bioblock Scientific) /ADN, au ratio 1:1, est formé en Opti Pro SFM (Invitrogen). Le complexe est ensuite déposé sur les cellules en suspension préalablement centrifugées et reprises à 1^{E}6 c/ml en FreeStyle CHO EM dans un cultiflask (Sartorius) (5ml) et incubé à 200 tr/min à 37°C, 8% de CO₂. Les surnageants sont récoltés à J+5 pour évaluation du taux de molécule sécrété dans le milieu.

Dans HEK293F, les PS sont évalués en transfection transitoire selon le protocole du kit FreeStyle (Invitrogen). Les cellules parentales sont ensemencées 24H avant la transfection (J-1) en Erlenmeyer à 6^{E}5 cv/ml en milieu F17 293 EM (Fisher Bioblock Scientific) et incubées à 120 tr/min 37°C, 8% CO₂. Le jour de la transfection un complexe 293Fectin/ADN, au ratio 2:1, est formé en Opti Pro SFM. Le complexe est ensuite déposé sur les cellules en suspension préalablement centrifugées et reprises à 1^{E}6 c/ml en F17 293 EM et incubé à 200 tr/min à 37°C, 8% de CO₂. Les surnageants sont récoltés à J+5 pour évaluation du taux de molécule sécrété dans le milieu.

### 1.5 Transfection stable

Les évaluations sont réalisées sur des pools de transfectants (« transfection en pool stable ») afin de comparer les différentes constructions sur la base d'un niveau d'expression moyenné sur un grand nombre de transfectants (plusieurs milliers).

Dans YB2/0, les transfections en pool stables sont réalisées de la façon suivante :
Les cellules doivent avoir une croissance stabilisée et décongelée depuis au moins 4 semaines en milieu EMS + 5% SVF dans des flask F150 (80ml). Les cellules sont repiquées la veille à 2^{E}5 cv/ml en milieu EMS + 5% SVF.

Le jour de l'électroporation, les cellules sont électroporées par Gene Pulser Xcell (BioRad) avec un voltage de 230V et une capacitance de 960µF dans des cuvettes (Biorad) de 4 mm avec 5^{E}6 cv (qsp 500µl de tampon d'électroporation du kit electrobuffer (Ozyme) contenant l'ADN plasmidique linéarisé). Après électroporation l'étalement est réalisé en plaques de 24 puits (P24) (25000 cellules/puits) en milieu EMS + 5% SVF.

A J+3 : Mise en milieu sélectif pour obtenir les concentrations finales suivantes : EMS + 5%SVF + G418 1g/1 + rouge de phénol 1%.

A J+7 : Renouvellement des plaques avec le milieu correspondant.

A J+10 : Lorsque les puits sont bien poussés, faire 3 pools de 8 puits P24, repiquer les cellules à 2^{E}5 cv/ml en F25 et réaliser une production maximale (Prod max à J+7), le surnageant et récolté et dosé en FastElysa.

Dans PER.C6, les cellules parentales utilisées sont des cellules PERC6SF adaptées en milieu Permab (ThermoFisher) et cultivées durant 3 semaines sous agitation à 100 tr/min. Deux jours avant électroporation, les cellules repiquées sont passées à 5^{E}5 cv/ml par un changement complet du milieu Permab. Le jour de l'électroporation, chaque pool est préparé par 5 électroporation dans des cuvettes de 4mm pour 6^{E}6 cv avec 8 µg d'ADN plasmidique linéarisé. Les cellules sont électroporées par Gene Pulser Xcell (BioRad) selon les instructions du fabricant.

48 heures après la transfection, la pression de sélection (G418 à 125 µg/ml) est ajoutée aux cellules qui sont maintenues en culture pendant environ 4 à 5 semaines en adaptant le volume de culture pour garder une concentration de 3^{E}5 cv/ml à chaque passage. Après un chute de viabilité cellulaire jusqu'à 20 %, les cellules sont passées en condition agitée. Lorsque la viabilité atteint environ 50 % et à 85 %, une évaluation de la productivité du pool est effectuée sur des cultures en batch à J+7.

Pour chaque construction, 3 pools différents sont effectués par lignée cellulaire et analysés. Les productions en pool sont exprimées en ng/ml.

### 1.6 Evaluation du taux de protéine recombinante sécrétée

L'évaluation du taux de chaine kappa libre de l'anticorps anti-Rh(D) T125 ainsi que la production d'IgG1 d'anti-CD20 ou d'anti- Rh(D) T12 sont déterminés par la technique Enzyme-Linked Immunosorbent Assay (ELISA).

La chaine kappa libre présente dans le surnageant de culture est capturée pendant 2h par un anticorps de chèvre anti-kappa humain (Caltag Lab) qui est adsorbé sur des plaques de 96puits. L'anticorps capturé est ensuite révélé par un anti-kappa humain de chèvre biotinylé (Pierce) puis ajout de streptavidine couplée à la peroxydase (Pierce). Entre chaque étape 4 lavages sont effectués pour éliminer les protéines pou réactifs n'entrant par dans le complexe. La révélation est faite par ajout du substrat de l'enzyme, l'PD (Sigma) et arrêt de la réaction par du HCl 1N. La lecture se fait au spectrophotomètre à 492nm. La concentration d'anticorps est déterminée en comparaison d'une gamme étalon.

Les IgG1 produites en transfection transitoire et stables sont évaluées par le kit Fast ELYSA (RD-biotech) selon les instructions du fournisseur. La lecture de la densité optique se fait au spectrophotomètre à 450nm. La concentration d'anticorps est déterminée en comparaison d'une gamme étalon contenue dans le kit.

La comparaison est effectuée entre la chaine kappa de l'anticorps anti-Rh(D) T125 sécrétée par les peptides signaux selon l'invention et celle sécrétée par son peptide signal naturel.

La comparaison est également effectuée entre l'anticorps anti-Rh(D) T125 ou l'anticorps anti-CD20 produit à l'aide d'au moins un des peptides signaux selon l'invention et celui produit par les peptides signaux naturels de chacun des anticorps respectivement.

Le peptide signal naturel de la chaine légère de l'anticorps anti-Rh(D) T125 est codé par l'acide nucléique SEQ ID NO: 30 (atgagggtccccgctcagctcctggggctcctgctgctctggctcccaggtgccagatgt).

Le peptide signal naturel de la chaine lourde de l'anticorps anti-Rh(D) T125 est codé par l'acide nucléique SEQ ID NO: 31 (atggagtttgggctgagctgggttttcctcgttgctcttttaagaggtgtccagtgt).

Le peptide signal naturel de la chaine légère de l'anticorps anti-CD20 est codé par l'acide nucléique SEQ ID NO : 32 (atggattttcaagtgcagattttcagcttcctgctaatcagtgcttcagtcataatgtccagagga).
Le peptide signal naturel de la chaine lourde de l'anticorps anti-CD20 est codé par l'acide nucléique SEQ ID NO : 33 (atgggattcagcaggatctttctcttcctcctgtcagtaactacaggtgtccactcc).

### Exemple 2 : Evaluation in vitro des peptides signaux sur la sécrétion d'une chaîne légère d'anticorps

Le pouvoir sécréteur de ces 7 peptides signaux est testé *in vitro* en transfection transitoire dans les lignées PER.C6, CHO-S, YB2/0. La méthode de transfection transitoire est décrite dans la partie ci-dessus (voir partie 1.2).

### 2.1 Effet de 7 peptides signaux dans la lignée PER.C6

Les résultats obtenus à partir des 4 transfections transitoires réalisées sur 4 semaines différentes permettent d'observer des différences significatives entre les PS.

Une comparaison multiple est réalisée pour les moyennes (ng/mL) de production de chaîne légère d'Ig obtenues avec les différents peptides signaux dans la lignée PER.C6 (Tableau 4). La méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

**Tableau 4**

| | *Effectif* | *Moyenne* | *Groupe homogène* |
|---|---|---|---|
| EPO_h | 16 | 3828,92 | X |
| PSK_CAT13 | 16 | 3834,16 | X |
| HAVT20 | 16 | 3985,15 | X |
| PSG_12G4 | 16 | 4468,87 | XX |
| PSG_5E5 | 16 | 5207,61 | XX |
| PSG D29 | 16 | 5563,95 | X |
| PSG_XXII49 | 16 | 5696,46 | X |

Deux groupes homogènes sont identifiés en utilisant des colonnes de X. Dans chaque colonne, les niveaux contenant des X forment un groupe de moyennes à l'intérieur desquelles il n'y a pas de différences statistiquement significatives. Les peptides signaux des chaînes lourdes des anticorps XXII49 et D29 sont significativement plus efficaces dans la production de la chaîne légère d'Ig (anti-Kh(D)) que les PS de l'anticorps Cat13 (anti-CD20), de l'HAVT20, et de l'EPO.

Les différences de production obtenues entre les différents PS sont ténues. Néanmoins, les PS des chaînes lourdes de l'anticorps XXII49 et de l'anticorps D29 se démarquent des autres dans la lignée PER.C6, avec un léger avantage pour celui de l'anticorps XXII49. Les trois moins bons peptides sont ceux de l'anticorps Cat13, de l'HAVT20, et de l'EPO.

### 2.2 Effet de 7 peptides signaux dans la lignée YB2/0

Les résultats obtenus à partir des 5 transfections réalisées sur 5 semaines différentes permettent d'observer des différences significatives entre les PS.

Une comparaison multiple est réalisée pour les moyennes (ng/mL) de production de chaîne légère d'Ig obtenues avec les différents peptides signaux dans la lignée YB2/0 (Tableau 5). La méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

**Tableau 5**

| | *Effectif* | *Moyenne* | *Groupe homogène* |
|---|---|---|---|
| PSK_CAT13 | 20 | 2744,26 | X |
| HAVT20 | 20 | 3181,69 | XX |
| EPO_h | 20 | 3479,19 | XXX |
| PSG_5E5 | 20 | 3756,33 | XX |
| PSG_D29 | 20 | 4255,05 | XX |
| PSG_12G4 | 20 | 5131,64 | XX |
| PSG_XXII49 | 20 | 5372,39 | X |

Cinq groupes homogènes sont identifiés en utilisant des colonnes de X. Le PS de la chaîne lourde de l'anticorps XXII49 est significativement plus efficace que tous les autres peptides mis à part celui du 12G4. En revanche, le PS de la chaîne légère de l'anti-CD20 (Catl3) apparaît comme le moins bon des PS avec ceux de l'HAVT20, et de l'EPO.

Les PS des chaînes lourdes des anticorps XXII49, 12G4, et D29 apparaissent comme les meilleurs PS avec un avantage à celui du XXII49. Les trois moins bons peptides sont ceux de l'anticorps Cat13, de l'HAVT20, et de l'EPO, comme observé précédemment dans la lignée PER.C6.

### 2.3 Effet de 7 peptides signaux dans la lignée CHO-S

Les résultats obtenus à partir des 4 transfections réalisées sur 4 semaines différentes permettent d'observer des différences significatives entre les PS.

Une comparaison multiple est réalisée pour les moyennes (ng/mL) de production de chaîne légère d'Ig obtenues avec les différents peptides signaux dans la lignée CHO-S (Tableau 6). La méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

**Tableau 6**

| | *Effectif* | *Moyenne* | *Groupe homogène* |
|---|---|---|---|
| EPO_h | 16 | 8210,98 | X |
| HAVT20 | 16 | 9297,38 | X |
| PSK_CAT13 | 16 | 10345,3 | XX |
| PSG_D29 | 16 | 10994,3 | XX |
| PSG_5E5 | 16 | 13399,1 | XX |
| PSG_12G4 | 16 | 14156,1 | X |
| PSG_XXII49 | 16 | 14720,1 | X |

Trois groupes homogènes sont identifiés en utilisant des colonnes de X. Le PS de la chaîne lourde de l'anticorps XXII49 est significativement plus efficace que ceux des anticorps D29, Cat13, de l'HAVT20 et de l'EPO. En revanche, son pouvoir de sécrétion est similaire à ceux des chaînes lourdes des anticorps 12G4 et 5E5.

Les PS des chaînes lourdes des anticorps XXII49, et 12G4, sont les meilleurs PS avec un avantage à celui du XXII49. Les trois moins bons peptides sont bien ceux de l'anticorps Cat13, de l'HAVT20, et de l'EPO, comme observé précédemment dans les lignées PER.C6, et YB2/0.

### Exemple 3 : Validation in vitro des peptides signaux sur la sécrétion d'une immunoglobuline entière

Les deux meilleurs PS identifiés (XXII49 et 12G4) *in silico* et *in vitro* ainsi qu'un PS artificiel selon l'invention, MB7, ont été testés pour évaluer le gain potentiel de productivité en anticorps entiers que pourraient apporter ces PS fusionnés aux chaînes lourde et légère d'immunoglobulines. Les anticorps testés sont l'anti-Rh(D) (R593) et l'anti-CD20 (R603). Les différentes molécules ont été testées en transfection transitoire dans les lignées PER.C6, YB2/0, CHO-S et HEK puis sur des pools stables dans PER.C6 et YB2/0.

La méthode de transfection transitoire ainsi que celle de transfection stable dans chaque lignée concernée sont décrites dans les parties susmentionnées (voir 1.4 et 1.5).

### 3.1 Effet des peptides signaux dans la lignée PER.C6

### 3.1.1 Transfections transitoires

Les résultats obtenus à partir des 4 transfections transitoires réalisées sur 4 semaines différentes permettent d'observer des différences significatives entre les PS.

La sécrétion moyenne de l'anticorps R593 à l'aide du peptide signal MB7 selon l'invention est augmentée d'un facteur 1,80 par rapport à celle de l'anticorps R593 lié à son propre peptide signal. Le peptide signal 12G4 et le peptide signal XXII49 permettent également d'augmenter respectivement d'un facteur de 1,71 et 1,58 le taux de sécrétion de l'anticorps R593 dans la lignée PER.C6. (figure 3 et tableau 7).

**Tableau 7**

| | *Effectif* | *Moyenne* | *Groupe homogène* |
|---|---|---|---|
| WT_R593 | 12 | 425,03 | X |
| XXII49_R593 | 12 | 672,51 | X |
| 12G4_R593 | 12 | 727,532 | XX |
| MB7_R593 | 11 | 766,072 | X |

Dans le tableau 7, la méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

### 3.1.2. Transfections tables

Les cellules PER.C6 ont été transfectées pour la génération de pools stables comme décrit à la section 1.5. Ces transfections ont été répétées sur 3 semaines pour être comparées aux résultats obtenus en transfection transitoire.

Les résultats sont montrés dans le tableau 8 et la figure 7.

Dans le tableau 8, la méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

**Tableau 8**

| | *Effectif* | *Moyenne* | *Groupe homogène* |
|---|---|---|---|
| WT_R603 | 6 | 1708,87 | X |
| MB7_R603 | 6 | 2293,53 | X |
| WT_R593 | 6 | 4576,45 | X |
| 12G4_R593 | 6 | 5258,34 | X |
| MB7_R593 | 6 | 5629,72 | X |

Une production en batch sur 7 jours a été effectuée sur les trois pools stables et les prélèvements ont été dosés par FastELYSA. Les résultats de la production à J+7 ont été analysés statistiquement pour comparer l'effet des peptides signaux sur la sécrétion d'IgG1. L'analyse statistique montre une différence significative entre le peptide signal MB7 et les peptides naturels (WT), tant pour l'anticorps anti-CD20 (R603) que pour l'anti-Rh(D) (R593) (tableau 8).

Pour l'anticorps anti-Rh(D), le peptide signal 12G4 (chaine lourde) montre également une moyenne significativement différente de celle des peptides signaux naturels (WT).

La figure 7 montre le rapport des productions moyennes entre les peptides signaux optimisés et des naturels. On observe un taux de production supérieur d'un facteur 1,3 pour le MB7-R603 et d'un facteur 1,2 pour le MB7-R593. Ceci montre donc que le peptide signal MB7 apporte un réel gain de productivité.

Pour l'anticorps anti-Rh(D) contenant le peptide signal 12G4, la différence de moyenne significative montrée par le test des étendues multiples se traduit par une augmentation de productivité d'un facteur 1,15.

L'effet des peptides signaux sur la viabilité cellulaire a également été étudié pour la production de l'anticorps anti-CD20(R603) (figure 8A) ou la production de l'anticorps anti-Rh(D)(R593) (figure 8B), dans la lignée cellulaire PER.C6. Pour l'anticorps anti-CD20 (R603), on observe que le recouvrement de la viabilité après transfection et ajout de la pression de sélection se fait légèrement plus rapidement en présence du peptide signal MB7 qu'en présence des peptides naturels. Ceci apporte un léger gain de temps dans l'établissement du pool stable car la remise en condition agitée des cellules contenant le MB7 peut se faire 1 à 2 jours avant les cellules contenant les peptides signaux naturels. L'anticorps anti-CD20 s'accumule dans la cellule et produit un effet toxique pour la cellule. Le peptide signal MB7, en augmentant la sécrétion de l'anticorps, diminue cet effet toxique et favorise donc ainsi la reprise des cellules. Cet effet ne s'observe pas avec l'anticorps anti-Rh(D) qui a une meilleure productivité que l'anti-CD20 et ne subit donc pas cette toxicité par accumulation de l'anticorps dans la cellule (figure 8B).

### 3.2 Effet des peptides signaux dans la lignée YB2/0

### 3.2.1 Transfections transitoires

Les résultats obtenus à partir des 3 transfections transitoires réalisées sur 3 semaines différentes permettent d'observer des différences significatives entre les PS (tableau 9 ).

**Tableau 9**

| | *Effectif* | *Moyenne* | *Groupe homogène* |
|---|---|---|---|
| 12G4_R603 | 12 | 618,756 | X |
| MB7_R603 | 12 | 656,243 | X |
| XXII49_R603 | 12 | 904,177 | X |
| WT_R603 | 12 | 374,04 | X |

Dans le tableau 9, la méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

La sécrétion moyenne de l'anticorps R603 à l'aide du peptide MB7 selon l'invention est augmentée d'un facteur 1,75 par rapport à celle de l'anticorps R603 lié à son propre peptide signal (figure 4). Le peptide signal 12G4 et le peptide signal XXII49 permettent également d'augmenter respectivement d'un facteur de 1,65 et 2,42 le taux de sécrétion de l'anticorps R603 dans la lignée YB2/0.

### 3.2.2 Transfections stables

Les cellules YB2/0 ont été transfectées pour la génération de pools stables comme décrit à la section 1.5. Ces transfections ont été répétées sur 3 semaines pour être comparées aux résultats obtenus en transfection transitoire.

Une production en batch sur 7 jours a été effectuée sur les trois pools stables et les prélèvements ont été dosés par FastELYSA. Les résultats de la production à J+7 ont été analysés statistiquement pour comparer l'effet des peptides signaux sur la sécrétion d'IgG1.

Le peptide signal MB7 donne le même niveau de productivité de l'anticorps R603 dans les cellules YB2/0 que celui de son peptide signal naturel.

### 3.3 Effet des peptides signaux dans la lignée CHO-S

Les résultats obtenus à partir des 3 transfections transitoires réalisées sur 3 semaines différentes permettent d'observer des différences significatives entre les PS (tableau 10).

**Tableau 10**

| | *Effectif* | *Moyenne* | *Groupe homogène* |
|---|---|---|---|
| XXII49_R593 | 12 | 2902,13 | X |
| 12G4_R593 | 10 | 2984,74 | X |
| MB7_R593 | 10 | 3570,06 | X |
| WT_R593 | 10 | 1702,78 | X |

Dans le tableau 10, la méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

La sécrétion moyenne de l'anticorps R593 à l'aide du peptide MB7 selon l'invention est augmentée d'un facteur 2,1 par rapport à celle de l'anticorps R593 lié à son propre peptide signal (figure 5). Le peptide signal 12G4 et le peptide signal XXII49 permettent également d'augmenter respectivement d'un facteur de 1,75 et 1,7 le taux de sécrétion de l'anticorps R593 dans la lignée CHO-S.

### 3.4 Effet des peptides signaux dans la lignée HEK293

Les résultats obtenus à partir des 3 transfections transitoires réalisées sur 3 semaines différentes permettent d'observer des différences significatives entre les PS (Tableau 11).

**Tableau 11**

| | *Effectif* | *Moyenne* | *Groupe homogène* |
|---|---|---|---|
| MB7_R593 | 12 | 1490,05 | X |
| XXII49_R593 | 12 | 1578,98 | X |
| 12G4_R593 | 12 | 1598,12 | X |
| WT_R593 | 12 | 1099,81 | X |

Dans le tableau 11, la méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

La sécrétion moyenne de l'anticorps R593 à l'aide du peptide MB7 selon l'invention est augmentée d'un facteur 1,35 par rapport à celle de l'anticorps R593 lié à son propre peptide signal (figure 6). Le peptide signal 12G4 et le peptide signal XXII49 permettent également d'augmenter respectivement d'un facteur de 1,45 et 1,44 le taux de sécrétion de l'anticorps R593 dans la lignée HEK 293.

### 3.5 Effet du changement de peptide signal sur la structure primaire de la molécule produite.

Afin de voir si le changement de peptide signal n'a pas d'impact sur la structure primaire de l'anticorps sécrété, des analyses physico chimiques par spectrométrie de masse et séquençage N-terminal par la technique d'Edman au niveau des chaines lourde et légère de l'anti-Rh(D) et l'anti-CD20 produits dans YB2/0 et PER.C6 en transfection stable ont été réalisés.

Les résultats de spectrométrie de masse résumés dans le tableau 12 montrent que les masses observées pour les 16 anticorps analysés correspondent aux masses théoriques attendues avec un clivage effectif du peptide signal.

La modification du peptide signal n'a donc pas d'impact sur la séquence protéique des anticorps produits.

**Tableau 12**

| **Anticorps** | | **Fc** | | **LC** | | **Fab** | |
|---|---|---|---|---|---|---|---|
| | | **Masse théorique (Da)** | **Masse expérimentale (Da)** | **Masse théorique (Da)** | **Masse expérimentale (Da)** | **Masse théorique (Da)** | **Masse expérimentale (Da)** |
| **R593 YB2/0 WT** | (H) | **25 058** | 25 058 | **23 495** | 23 496 | **26 370** | 26 372 |
| R593 YB2/0 AMHRII | (E) | **25 058** | 25 058 | **23 495** | 23 497 | **26 370** | 26 372 |
| R593 YB2/0 MB7 | (F) | **25 058** | 25 058 | **2 495** | 23 497 | **26 370** | 26 372 |
| R593 YB2/0 XXII49 | (G) | **25 058** | 25 058 | **23 495** | 23 496 | **26 370** | 26 372 |
| | | | | | | | |
| **R593 PER.C6 WT** | (M) | **25 366** | 25 367 | **23 495** | 23 496 | **26 310** | 26 371 |
| R593 PER C6 AMHRII | (O) | **25 366** | 25 367 | **23 495** | 23 497 | **26 370** | 26 372 |
| R593 PER.C6 MB7 | (N) | **25 366** | 25 366 | **23 495** | 23 496 | **26 370** | 26 371 |
| R593 PER.C6 XXII49 | (P) | **25 366** | 25 367 | **23 495** | 23 496 | **26 370** | 26 372 |
| | | | | | | | |
| **R603 YB2/0 WT** | (D) | **25 058** | 25 058 | **23 168** | 23 170 | **25 197** | 25 200 |
| R603 YB2/0 AMHRII | (A) | **25 058** | 25 059 | **23 168** | 23 170 | **25 197** | 25 200 |
| R603 YB2/0 MB7 | (B) | **25 058** | 25 059 | **23 168** | 23 170 | **25197** | 25 200 |
| R603 YB2/0 XXII49 | (C) | **25 058** | 25 059 | **23 168** | 23 170 | **25 197** | 25 200 |
| | | | | | | | |
| **R603 PER.C6 WT** | (I) | **25 366** | 25 367 | **23 168** | 23 170 | **25 197** | 25 199 |
| R603 PER.C6 AMHRII | (K) | **25 366** | 25 367 | **23 168** | 23 169 | **25 197** | 25 199 |
| R603 PER.C6 MB7 | (J) | **25 366** | 25 367 | **23 168** | 23 169 | **25 197** | 25 199 |
| R603 PER.C6 XXII49 | (L) | **25 366** | 25 367 | **23 168** | 23 169 | **25 197** | 25 199 |

## Revendications

1. Utilisation d'un peptide signal pour la production d'un polypeptide recombinant d'intérêt dans un système d'expression, ledit peptide signal comprenant au moins 12 acides aminés de formule (I) :
(X1)ᵢX2X3X4SX5X6X7,
dans laquelle :
- X1 est un peptide contenant de 3 à 6 acides aminés, i égal à 0 ou 1,
- X2 est un peptide contenant de 3 à 9 acides aminés hydrophobes choisis parmi Gly, Ala, Val, Ile, Pro, Phe, Trp,
- X3 est un peptide contenant de 3 à 5 acides aminés, ledit peptide comprenant au moins 3 leucines, contigües,
- X4 est un peptide contenant de 2 à 5 acides aminés choisis parmi Ala, Thr, Ser, Gln, Ile, Met,
- X5 est Ala ou Val,
- X6 est Gln, Asn ou His,
- X7 est Ala ou Cys,
sous réserve que :
- lorsque ledit peptide signal est issu d'un précurseur naturel d'une protéine déterminée, ledit polypeptide d'intérêt est différent de ladite protéine, n'importe quel polypeptide d'intérêt pouvant être lié à l'un desdits peptides signaux pour être sécrété dans le milieu extracellulaire.

2. Utilisation selon la revendication 1, dans laquelle ledit peptide signal universel est représenté par la formule (II) :
M(X1)iX2X3X4SX5X6X7,
dans laquelle X1, i, X2, X3 X4, X5 X6 et X7 ont les significations indiquées à la revendication 1.

3. Utilisation selon la revendication 1, dans laquelle :
- X1, X2 et X4 ont les significations indiquées à la revendication 1,
- i = 1,
- X3 est un peptide constitué de leucines, représenté par Ln, dans lequel n est le nombre de leucines, n étant un nombre supérieur ou égal à 3,
- X4 est un peptide contenant de 2 à 5 acides aminés choisis parmi Ala, Thr, Ser, Gln, Ile,
- X5 est Ala,
- X6 est Gln ou Asn,
- X7 est Ala,
ledit peptide comprenant la séquence d'acides aminés suivante: X1X2LnX4SAX6A.

4. Utilisation d'un peptide signal selon l'une quelconque des revendications 1 à 3, dans laquelle le système d'expression est une lignée cellulaire d'eucaryote supérieur, et notamment une lignée cellulaire choisie parmi SP2/0, SP2/0-Ag 14, NS0, autres myélomes de rat comme IR983F, des lignées humaines comme Namalwa, Wil-2, Jurkat, Molt-4, PER.C6, HEK293T/17, HEK293, HEK-293.2, Vero, Cos-1 ou Cos-7, BHK, CHO-K-1, CHO-Lec1, CHO-Lec10, CHO-Lec13, CHO Pro-5, CHO DX B11 et CHO DG44 et autres lignées comme EBx avec notamment EB66K6H6, et P3X63Ag8.653, YB2/0, CHO-S et HEK-293F.

5. Utilisation d'un peptide signal selon l'une quelconque des revendications 1 à 4, dans laquelle le système d'expression est un animal transgénique, choisis parmi chèvres, moutons, bisons, chameaux, vaches, cochons, lapins, chevaux, rats, souris ou lamas.

6. Utilisation selon l'une quelconque des revendications 1 à 5, dans laquelle le polypeptide recombinant d'intérêt est choisi parmi une hormone, une enzyme, une chaîne d'immunoglobuline, une immunoglobuline entière ou tout fragment dérivé d'une immunoglobuline, une protéine intervenant dans la réaction immunitaire tels que cytokines, interleukines, facteurs du complément, une protéine chimérique, ou d'autres protéines thérapeutiques telle que des facteurs de coagulation, des protéines de la matrice extracellulaire, ou des récepteurs solubles.

7. Peptide signal comprenant une séquence d'au moins 12 acides aminés de formule III:
X1X2X3X4SX5X6X7,
dans laquelle :
- X1 est un peptide contenant de 3 à 6 acides aminés,
- X2 est un peptide contenant de 3 à 9 acides aminés hydrophobes choisis parmi Gly, Ala, Val, Ile, Pro, Phe, Trp,
- X3 est un peptide contenant de 3 à 5 acides aminés, ledit peptide comprenant au moins 3 leucines contigües,
- X4 est un peptide contenant de 2 à 5 acides aminés choisis parmi Ala, Thr, Ser, Gln, Ile,
- X5 est Ala,
- X6 est Gln ou Asn,
- X7 est Ala.

8. Peptide signal de formule (IV) :
M X1X2X3X4SX5X6X7,
dans laquelle X1, X2, X3 X4, X5 X6 et X7 ont les significations indiquées à la revendication 7.

9. Peptide signal de formule III selon la revendication 7 ou de formule (IV) selon la revendication 8, dans lequel :
- X3 est un peptide contenant 4 leucines contigües,
- le peptide X2 est WIF,
- X1, X4 et X6 ont les significations indiquées à la revendication 7,
le peptide signal répondant à la formule suivante : X1WIFX3X4SAX6A.

10. Peptide signal selon la revendication 7 à 9, dans lequel le peptide signal de formule (IV) comprend ou consiste en la séquence d'acides aminés : MRWSWIFLLLLSITSANA.

11. Acide nucléique codant l'un des peptides signaux selon l'une quelconque des revendications 7 à 10, notamment un peptide représenté par la séquence suivante : MRWSWIFLLLLSITSANA.

12. Précurseur d'un polypeptide recombinant d'intérêt, dans lequel un peptide signal comprend au moins 12 acides aminés de formule (I) : (X1)ᵢX2X3X4SX5X6X7, dans laquelle :
- X1 est un peptide contenant de 3 à 6 acides aminés, i égal à 0 ou 1,
- X2 est un peptide contenant de 3 à 9 acides aminés hydrophobes choisis parmi Gly, Ala, Val, Ile, Pro, Phe, Trp,
- X3 est un peptide contenant de 3 à 5 acides aminés, ledit peptide comprenant au moins 3 leucines contigües,
- X4 est un peptide contenant de 2 à 5 acides aminés choisis parmi Ala, Thr, Ser, Gln, Ile, Met,
- X5 est Ala ou Val,
- X6 est Gln, Asn ou His,
- X7 est Ala ou Cys,
sous réserve que lorsque ledit peptide signal est issu d'un précurseur naturel d'une protéine déterminée, ledit polypeptide d'intérêt est différent de ladite protéine,
n'importe quel polypeptide d'intérêt pouvant être lié à l'un desdits peptides signaux pour être sécrété dans le milieu extracellulaire, et notamment dans lequel le peptide signal est l'un des peptides signaux selon l'une quelconque des revendications 7 à 10.

13. Acide nucléique codant l'un des précurseurs d'un polypeptide recombinant d'intérêt selon la revendication 12.

14. Vecteur contenant un acide nucléique selon la revendication 13.

15. Procédé de production d'un polypeptide recombinant d'intérêt, comprenant :
- l'ajout d'un acide nucléique codant un peptide signal comprenant au moins 12 acides aminés de formule (I) :
(X1)ᵢX2X3X4SX5X6X7,
dans laquelle :
- X1 est un peptide contenant de 3 à 6 acides aminés, i égal à 0 ou 1,
- X2 est un peptide contenant de 3 à 9 acides aminés hydrophobes choisis parmi Gly, Ala, Val, Ile, Pro, Phe, Trp,
- X3 est un peptide contenant de 3 à 5 acides aminés, ledit peptide comprenant au moins 3 leucines, contigües,
- X4 est un peptide contenant de 2 à 5 acides aminés choisis parmi Ala, Thr, Ser, Gln, Ile, Met,
- X5 est Ala ou Val,
- X6 est Gln, Asn ou His,
- X7 est Ala ou Cys,
à l'extrémité 5' d'un acide nucléique codant un polypeptide recombinant d'intérêt, pour obtenir un acide nucléique codant le précurseur du susdit polypeptide recombinant d'intérêt, sous réserve que lorsque ledit peptide signal est issu d'un précurseur naturel d'une protéine déterminée, ledit polypeptide d'intérêt est différent de ladite protéine,
- le clonage de l'acide nucléique artificiel obtenu dans l'étape précédente à un vecteur d'expression, pour obtenir un vecteur capable d'exprimer le précurseur du susdit polypeptide recombinant d'intérêt, et
- la transfection d'une lignée cellulaire d'eucaryote supérieur par le vecteur d'expression comprenant ladite séquence nucléotidique artificielle et l'expression de ladite séquence nucléotidique,
- la récupération du polypeptide recombinant d'intérêt sécrété dans le milieu de culture.

## Patentansprüche

1. Verwendung eines Signalpeptids zur Herstellung eines interessierenden rekombinanten Polypeptids in einem Expressionssystem, wobei das Signalpeptid mindestens 12 Aminosäuren mit der folgenden Formel (I) umfasst:
(X1) ᵢX2X3X4SX5X6X7,
worin:
- X1 für ein Peptid steht, das 3 bis 6 Aminosäuren enthält, i für 0 oder 1 steht,
- X2 für ein Peptid steht, das 3 bis 9 hydrophobe Aminosäuren enthält, ausgewählt aus Gly, Ala, Val, Ile, Pro, Phe, Trp,
- X3 für ein Peptid steht, das 3 bis 5 Aminosäuren enthält, wobei das Peptid mindestens 3 aufeinander folgende Leucine enthält,
- X4 für ein Peptid steht, das 2 bis 5 Aminosäuren enthält, ausgewählt aus Ala, Thr, Ser, Gln, Ile, Met,
- X5 für Ala oder Val steht,
- X6 für Gln, Asn oder His steht,
- X7 für Ala oder Cys steht,
unter der Bedingung, dass:
- wenn das Signalpeptid aus einem natürlichen Vorläufer eines bestimmten Proteins stammt, das interessierende Polypeptid von dem Protein verschieden ist, unabhängig davon, welches interessierende Polypeptid an eines der Signalpeptide gebunden sein könnte, um in das extrazelluläre Medium sezerniert zu werden.

2. Verwendung nach Anspruch 1, wobei das universelle Signalpeptid durch die folgende Formel (II) dargestellt wird:
M(X1)iX2X3X4SX5X6X7,
worin X1, i, X2, X3, X4, X5, X6 und X7 die in Anspruch 1 angegebenen Bedeutungen aufweisen.

3. Verwendung nach Anspruch 1, worin:
- X1, X2 und X4 die in Anspruch 1 angegebenen Bedeutungen aufweisen,
- i = 1,
- X3 für ein Peptid steht, das aus Leucinen besteht, die durch Ln dargestellt werden, worin n für die Anzahl an Leucinen steht, wobei n für eine ganze Zahl größer oder gleich 3 steht,
- X4 für ein Peptid steht, das 2 bis 5 Aminosäuren enthält, ausgewählt aus Ala, Thr, Ser, Gln, Ile,
- X5 für Ala steht,
- X6 für Gln oder Asn steht,
- X7 für Ala steht,
wobei das Peptid die folgende Aminosäuresequenz umfasst: X1X2LnX4SAX6A.

4. Verwendung eines Signalpeptids nach einem der Ansprüche 1 bis 3, wobei das Expressionssystem eine Zelllinie höherer Eukaryoten ist, und insbesondere eine Zelllinie, ausgewählt aus SP2/0, SP2/0-Ag 14, NS0, anderen Rattenmyelomen, wie IR983F, humanen Linien, wie Namalwa, Wi1-2, Jurkat, Molt-4, PER.C6, HEK293T/17, HEK293, HEK-293.2, Vero, Cos-1 oder Cos-7, BHK, CHO-K-1, CHO-Lec1, CHO-Lec10, CHO-Lec13, CHO Pro-5, CHO DX B11 und CHO DG44, und anderen Linien, wie EBx, wie insbesondere EB66K6H6 und P3X63Ag8.653, YB2/0, CHO-S und HEK-293F.

5. Verwendung eines Signalpeptids nach einem der Ansprüche 1 bis 4, wobei es sich bei dem Expressionssystem um ein transgenes Tier handelt, ausgewählt aus Ziegen, Schafen, Wisenten/Bisons, Kamelen, Kühen, Schweinen, Kaninchen, Pferden, Ratten, Mäusen oder Lamas.

6. Verwendung nach einem der Ansprüche 1 bis 5, wobei das interessierende rekombinante Polypeptid ausgewählt ist aus einem Hormon, einem Enzym, einer Immunglobulinkette, einem vollständigen Immunglobulin oder jedem Fragment, das von einem Immunglobulin abgeleitet ist, einem Protein, das an der Immunreaktion beteiligt ist, wie beispielsweise Cytokine, Interleukine, Komplementfaktoren, einem chimären Protein oder anderen therapeutischen Proteinen, wie beispielsweise Koagulationsfaktoren, Proteinen der extrazellulären Matrix oder löslichen Rezeptoren.

7. Signalpeptid, das eine Sequenz aus mindestens 12 Aminosäuren mit der folgenden Formel III umfasst:
X1X2X3X4SX5X6X7,
worin:
- X1 für ein Peptid steht, das 3 bis 6 Aminosäuren enthält,
- X2 für ein Peptid steht, das 3 bis 9 hydrophobe Aminosäuren enthält, ausgewählt aus Gly, Ala, Val, Ile, Pro, Phe, Trp,
- X3 für ein Peptid steht, das 3 bis 5 Aminosäuren enthält, wobei das Peptid mindestens 3 aufeinander folgende Leucine enthält,
- X4 für ein Peptid steht, das 2 bis 5 Aminosäuren enthält, ausgewählt aus Ala, Thr, Ser, Gln, Ile,
- X5 für Ala steht,
- X6 für Gln oder Asn steht,
- X7 für Ala steht.

8. Signalpeptid mit der folgenden Formel (IV):
MX1X2X3X4SX5X6X7,
worin X1, X2, X3, X4, X5, X6 und X7 die in Anspruch 7 angegebenen Bedeutungen aufweisen.

9. Signalpeptid mit der Formel III nach Anspruch 7 oder mit der Formel (IV) nach Anspruch 8, worin:
- X3 für ein Peptid steht, das 4 aufeinander folgende Leucine enthält,
- das Peptid X2 für WIF steht,
- X1, X4 und X6 die in Anspruch 7 angegebenen Bedeutungen aufweisen,
das Signalpeptid der folgenden Formel entspricht:Xl WIFX3X4SAX6A.

10. Signalpeptid nach Anspruch 7 bis 9, wobei das Signalpeptid der Formel (IV) die folgende Aminosäuresequenz umfasst oder aus dieser besteht: MRWSWIFLLLLSITSANA.

11. Nukleinsäure, die eines der Signalpeptide nach einem der Ansprüche 7 bis 10 kodiert, insbesondere ein Peptid, das durch die folgende Sequenz dargestellt wird: MRWSWIFLLLLSITSANA.

12. Vorläufer eines interessierenden rekombinanten Polypeptids, worin ein Signalpeptid mindestens 12 Aminosäuren mit der folgenden Formel (I) umfasst: (X1)iX2X3X4SX5X6X7, worin:
- X1 für ein Peptid steht, das 3 bis 6 Aminosäuren enthält, i für 0 oder 1 steht,
- X2 für ein Peptid steht, das 3 bis 9 hydrophobe Aminosäuren enthält, ausgewählt aus Gly, Ala, Val, Ile, Pro, Phe, Trp,
- X3 für ein Peptid steht, das 3 bis 5 Aminosäuren enthält, wobei das Peptid mindestens 3 aufeinander folgende Leucine enthält,
- X4 für ein Peptid steht, das 2 bis 5 Aminosäuren enthält, ausgewählt aus Ala, Thr, Ser, Gln, Ile, Met,
- X5 für Ala oder Val steht,
- X6 für Gln, Asn oder His steht,
- X7 für Ala oder Cys steht,
unter der Bedingung, dass wenn das Signalpeptid aus einem natürlichen Vorläufer eines bestimmten Proteins stammt, das interessierende Polypeptid von dem Protein verschieden ist,
unabhängig davon, welches interessierende Polypeptid an eines der Signalpeptide gebunden sein könnte, um in das extrazelluläre Medium sezerniert zu werden, und wobei das Signalpeptid insbesondere eines der Signalpeptide nach einem der Ansprüche 7 bis 10 ist.

13. Nukleinsäure, die einen der Vorläufer eines interessierenden rekombinanten Polypeptids nach Anspruch 12 kodiert.

14. Vektor, der eine Nukleinsäure nach Anspruch 13 enthält.

15. Verfahren zur Herstellung eines interessierenden rekombinanten Polypeptids, das Folgendes umfasst:
- Anfügen einer Nukleinsäure, die ein Signalpeptid kodiert, das mindestens 12 Aminosäuren mit der folgenden Formel (I) umfasst:
(X1)iX2X3X4SX5X6X7,
worin:
- X1 für ein Peptid steht, das 3 bis 6 Aminosäuren enthält, i für 0 oder 1 steht,
- X2 für ein Peptid steht, das 3 bis 9 hydrophobe Aminosäuren enthält, ausgewählt aus Gly, Ala, Val, Ile, Pro, Phe, Trp,
- X3 für ein Peptid steht, das 3 bis 5 Aminosäuren enthält, wobei das Peptid mindestens 3 aufeinander folgende Leucine enthält,
- X4 für ein Peptid steht, das 2 bis 5 Aminosäuren enthält, ausgewählt aus Ala, Thr, Ser, Gln, Ile, Met,
- X5 für Ala oder Val steht,
- X6 für Gln, Asn oder His steht,
- X7 für Ala oder Cys steht,
an das 5'-Ende einer Nukleinsäure, die ein interessierendes rekombinantes Polypeptid kodiert, um eine Nukleinsäure zu erhalten, die den Vorläufer des oben genannten interessierenden rekombinanten Polypeptids kodiert, unter der Bedingung, dass wenn das Signalpeptid aus einem natürlichen Vorläufer eines bestimmten Proteins stammt, das interessierende Polypeptid von dem Protein verschieden ist,
- Klonieren der im vorherigen Schritt erhaltenen künstlichen Nukleinsäure mit einem Expressionsvektor, um einen Vektor zu erhalten, der in der Lage ist, den Vorläufer des oben genannten interessierenden rekombinanten Polypeptids zu exprimieren, und
- Transfizieren einer Zelllinie höherer Eukaryoten mit dem Expressionsvektor, der die künstliche Nukleotidsequenz umfasst, und Exprimieren der Nukleotidsequenz,
- Gewinnen des interessierenden rekombinanten Polypeptids, das in das Kulturmedium sezerniert wird.

## Claims

1. Use of a signal peptide for the production of a recombinant polypeptide of interest in an expression system, said signal peptide comprising at least 12 amino acids of formula (I) :
(X1)ᵢX2X3X4SX5X6X7
in which :
- X1 is a peptide containing from 3 to 6 amino acids, i equal to 0 or 1,
- X2 is a peptide containing from 3 to 9 hydrophobic amino acids chosen from Gly, Ala, Val, Ile, Pro, Phe, Trp,
- X3 is a peptide containing from 3 to 5 amino acids, said peptide comprising at least 3 contiguous leucines,
- X4 is a peptide containing from 2 to 5 amino acids chosen from Ala, Thr, Ser, Gln, Ile, Met,
- X5 is Ala or Val,
- X6 is Gln, Asn, or His,
- X7 is Ala or Cys,
provided that:
- when said signal peptide originates from a natural precursor of a determined protein, said polypeptide of interest is different from said protein, any polypeptide of interest being liable to be bound to one of said signal peptides in order to be secreted in the extracellular medium.

2. Use according to claim 1, in which said universal signal peptide is represented by the formula (II) :
M(X1)ᵢX2X3X4SX5X6X7,
in which X1, i, X2, X3, X4, X5, X6 and X7 have the meanings indicated in claim 1.

3. Use according to claim 1, in which :
- X1, X2 et X4 have the meanings indicated in claim 1,
- i = 1,
- X3 is a peptide constituted by leucines, represented by Ln, in which n is the number of leucines, n being a number greater than or equal to 3,
- X4 is a peptide containing from 2 to 5 amino acids chosen from Ala, Thr, Ser, Gln, Ile,
- X5 is Ala,
- X6 is Gln or Asn,
- X7 is Ala,
said peptide comprising the following sequence of amino acids : X1X2LnX4SAX6A.

4. Use of a signal peptide according to any one of claims 1 to 3, in which the expression system is a higher eukaryotic cell line, and in particular a cell line chosen from SP2/0, SP2/0-Ag 14, NSO, other rat myelomas such as IR983F, human lines such as Namalwa, Wil-2, Jurkat, Molt-4, PER.C6, HEK293T/17, HEK293, HEK-293.2, Vero, Cos-1 or Cos-7, BHK, CHO-K-1, CHO-Lec1, CHO-Lec10, CHO-Lec13, CHO Pro-5, CHO DX B11 and CHO DG44 and other lines such as EBx with in particular EB66K6H6, and P3X63Ag8.653, YB2/0, CHO-S and HEK-293F.

5. Use of signal peptide according to any one of claims 1 to 4, in which the expression system is a transgenic animal, chosen from goats, sheep, bisons, camels, cows, pigs, rabbits, horses, rats, mice or llamas.

6. Use according to any one of claims 1 to 5, in which the recombinant polypeptide of interest is chosen from a hormone, an enzyme, an immunoglobulin chain, a whole immunoglobulin or any fragment derived from an immunoglobulin, a protein involved in the immune response such as cytokines, interleukins, complement factors, a chimeric protein, or other therapeutic proteins such as coagulation factors, extracellular matrix proteins, or soluble receptors.

7. Signal peptide comprising a sequence of at least 12 amino acids of formula III :
X1X2X3X4SX5X6X7,
in which :
- X1 is a peptide containing from 3 to 6 amino acids,
- X2 is a peptide containing from 3 to 9 hydrophobic amino acids chosen from Gly, Ala, Val, Ile, Pro, Phe, Trp,
- X3 is a peptide containing from 3 to 5 amino acids, said peptide comprising at least 3 contiguous leucines,
- X4 is a peptide containing from 2 to 5 amino acids chosen from Ala, Thr, Ser, Gln, Ile,
- X5 is Ala,
- X6 is Gln or Asn
- X7 is Ala.

8. Signal peptide of formula (IV) :
M X1X2X3X4SX5X6X7,
in which X1,X2,X3,X4,X5,X6 and X7 have the meanings indicated in claim 7.

9. Signal peptide of formula III according to claim 7 or of formula (IV) according to claim 8, in which :
- X3 is a peptide containing 4 contiguous leucines,
- the peptide X2 is WIF,
- X1, X4, and X6 have the meanings indicated in claim 7,
the signal peptide being represented by the following sequence : X1WIFX3X4SAX6A.

10. Signal peptide according to claims 7 to 9, in which the signal peptide of formula (IV) comprises or consists of the sequence of amino acids: MRWSWIFLLLLSITSANA.

11. Nucleic acid encoding one of the signal peptides according to any one of claims 7 to 10, in particular a peptide represented by the following sequence: MRWSWIFLLLLSITSANA.

12. Precursor of a recombinant polypeptide of interest, in which a signal peptide comprises at least 12 amino acids of formula (I) : (X1)ᵢX2X3X4SX5X6X7, in which :
- X1 is a peptide containing from 3 to 6 amino acids, i equal to 0 or 1,
- X2 is a peptide containing from 3 to 9 hydrophobic amino acids chosen from Gly, Ala, Val, Ile, Pro, Phe, Trp,
- X3 is a peptide containing from 3 to 5 amino acids, said peptide comprising at least 3 contiguous leucines,
- X4 is a peptide containing from 2 to 5 amino acids chosen from Ala, Thr, Ser, Gln, Ile, Met,
- X5 is Ala or Val,
- X6 is Gln, Asn or His,
- X7 is Ala or Cys,
provided that when said signal peptide originates from a natural precursor of a determined protein, said polypeptide of interest is different from said protein, any polypeptide of interest being liable to be bound to one of said signal peptides in order to be secreted in the extracellular medium, and in particular in which the signal peptide is one of signal peptides according to any one of claims 7 to 10.

13. Nucleic acid encoding one of the precursors of a recombinant polypeptide of interest according to claim 12.

14. Vector containing a nucleic acid according to claim 13.

15. Method for producing a recombinant polypeptide of interest, comprising :
- the addition of a nucleic acid encoding a signal peptide comprising at least 12 amino acids of formula (I) : (X1)ᵢX2X3X4SX5X6X7,
in which :
- X1 is a peptide containing from 3 to 6 amino acids, i equal to 0 or 1,
- X2 is a peptide containing from 3 to 9 hydrophobic amino acids chosen from Gly, Ala, Ile, Pro, Phe, Trp,
- X3 is a peptide containing from 3 to 5 amino acids, said peptide comprising at least 3 contiguous leucines,
- X4 is a peptide containing from 2 to 5 amino acids chosen from Ala, Thr, Ser, Gln, Ile, Met,
- X5 is Ala or Val,
- X6 is Gln, Asn, or His,
- X7, is Ala or Cys,
to the 5' end of a nucleic acid encoding a recombinant polypeptide of interest, in order to obtain a nucleic acid encoding for the precursor of the abovementioned recombinant polypeptide of interest, provided that when said signal peptide originates from a natural precursor of a specific protein, said polypeptide of interest is different from said protein,
- the cloning of the artificial nucleic acid obtained in the previous stage to an expression vector, in order to obtain a vector capable of expressing the precursor of the abovementioned recombinant polypeptide of interest, and
- the transfection of a higher eukaryotic cell line by the expression vector comprising said artificial nucleotide sequence and the expression of said nucleotide sequence,
- the recovery of the recombinant polypeptide of interest secreted in the culture medium.


### SEQUENCE LISTING

<110> LFB Biotechnologies
<120> NOUVEAU PEPTIDE SIGNAL, ET SON UTILISATION POUR LA PRODUCTION DE PROTEINES RECOMBINANTES
<130> WOB 10 AA LFB PEPS
<150> FRn ° 10/51905 <151> 2010-03-17
<160> 33
<170> PatentIn version 3.5
<210> 1
   <211> 18
   <212> PRT
   <213> MB7 peptide signal
<400> 1
   Met Arg Trp Ser Trp Ile Phe Leu Leu Leu Leu Ser Ile Thr Ser Ala
   1               5                   10                  15
   Asn Ala
<210> 2
   <211> 19
   <212> PRT
   <213> 12G4 peptide signal
<400> 2
   Met Arg Trp Ser Trp Ile Phe Leu Phe Leu Leu Ser Ile Thr Ala Ser
   1               5                   10                  15
   Val His Cys
<210> 3
   <211> 19
   <212> PRT
   <213> XXII49 peptide signal
<400> 3
   Met Ala Trp Val Trp Thr Leu Leu Phe Leu Met Ala Ala Ala Gln Ser
   1               5                   10                  15
   Ala Gln Ala
<210> 4
   <211> 54
   <212> DNA
   <213> MB7 acide nucléique
<400> 4
   atgcgatgga gctggatctt cctgctgctg ctgagcatca ccagcgccaa cgcc 54
<210> 5
   <211> 40
   <212> DNA
   <213> P1_12G4
<400> 5
   ctcttgctag cgccgccacc atgcgatgga gctggatctt 40
<210> 6
   <211> 40
   <212> DNA
   <213> P2_12G4
<400> 6
   cacttgcagt tattgacagg aggaagagaa agatccagct 40
<210> 7
   <211> 40
   <212> DNA
   <213> P3_12G4
<400> 7
   actgcaagtg tccattgcgc catccggatg acccagtctc 40
<210> 8
   <211> 40
   <212> DNA
   <213> P1_XXII49
<400> 8
   ctcttgctag cgccgccacc atggcttggg tgtggacctt 40
<210> 9
   <211> 40
   <212> DNA
   <213> P2_XXII49
<400> 9
   cactttgggc agctgccatc aggaatagca aggtccacac 40
<210> 10
   <211> 40
   <212> DNA
   <213> P3_XXII49
<400> 10
   gcccaaagtg cccaagcagc catccggatg acccagtctc 40
<210> 11
   <211> 40
   <212> DNA
   <213> P1_D29
<400> 11
   ctcttgctag cgccgccacc atggagcttg ggctgagctg 40
<210> 12
   <211> 41
   <212> DNA
   <213> P2_D29
<400> 12
   acacctctta aaagagcaac gagaaaaacc cagctcagcc c 41
<210> 13
   <211> 40
   <212> DNA
   <213> P3_D29
<400> 13
   ttaagaggtg tccagtgtgc catccggatg acccagtctc 40
<210> 14
   <211> 40
   <212> DNA
   <213> P1_5E5
<400> 14
   ctcttgctag cgccgccacc atgggttgga gctgtatcat 40
<210> 15
   <211> 42
   <212> DNA
   <213> P2_5E5
<400> 15
   acacctgtag ctgttgctac cagaaagaag atgatacagc tc 42
<210> 16
   <211> 41
   <212> DNA
   <213> P3_5E5
<400> 16
   agctacaggt gtgcactccg ccatccggat gacccagtct c 41
<210> 17
   <211> 44
   <212> DNA
   <213> P1_CAT13
<400> 17
   ctcttgctag cgccgccacc atggattttc aagtgcagat tttc 44
<210> 18
   <211> 45
   <212> DNA
   <213> P2_CAT13
<400> 18
   cattatgact gaagcactga ttagcaggaa gctgaaaatc tgcac 45
<210> 19
   <211> 45
   <212> DNA
   <213> P3_CAT13
<400> 19
   cttcagtcat aatgtccaga ggagccatcc ggatgaccca gtctc 45
<210> 20
   <211> 50
   <212> DNA
   <213> P1_EPO(H)
<400> 20
   ctcttgctag cgccgccacc atgggggtgc acgaatgtcc tgcctggctg 50
<210> 21
   <211> 46
   <212> DNA
   <213> P2_EPO(H)
<400> 21
   cagagggagc gacagcaggg acaggagaag ccacagccag gcagga 46
<210> 22
   <211> 52
   <212> DNA
   <213> P3_EPO(H)
<400> 22
   tcgctccctc tgggcctccc agtcctgggc gccatccgga tgacccagtc tc 52
<210> 23
   <211> 42
   <212> DNA
   <213> P1_HAVT20
<400> 23
   ctcttgctag cgccgccacc atggcatgcc ctggcttcct gt 42
<210> 24
   <211> 43
   <212> DNA
   <213> P2_HAVT20
<400> 24
   aattcaagac aggtggagat cacaagtgcc cacaggaagc cag 43
<210> 25
   <211> 45
   <212> DNA
   <213> P3_HAVT20
<400> 25
   cctgtcttga attttccatg gctgccatcc ggatgaccca gtctc 45
<210> 26
   <211> 42
   <212> DNA
   <213> P2_KAPPA_Xba1
<400> 26
   gcgagctcta gagttcacta acactctccc ctgttgaagc tc 42
<210> 27
   <211> 20
   <212> DNA
   <213> 2BGHPA
<400> 27
   cagatggctg gcaactagaa 20
<210> 28
   <211> 57
   <212> DNA
   <213> 12G4 acide nucléique
<400> 28
   atgcgatgga gctggatctt tctcttcctc ctgtcaataa ctgcaagtgt ccattgc 57
<210> 29
   <211> 57
   <212> DNA
   <213> XXII49 acide nucléique
<400> 29
   atggcttggg tgtggacctt gctattcctg atggcagctg cccaaagtgc ccaagca 57
<210> 30
   <211> 60
   <212> DNA
   <213> R 593 VL peptide signal
<400> 30
   atgagggtcc ccgctcagct cctggggctc ctgctgctct ggctcccagg tgccagatgt 60
<210> 31
   <211> 57
   <212> DNA
   <213> R 593 VH peptide signal
<400> 31
   atggagtttg ggctgagctg ggttttcctc gttgctcttt taagaggtgt ccagtgt 57
<210> 32
   <211> 66
   <212> DNA
   <213> R603 VL peptide signal
<400> 32
   atggattttc aagtgcagat tttcagcttc ctgctaatca gtgcttcagt cataatgtcc 60
agagga 66
<210> 33
   <211> 57
   <212> DNA
   <213> R603 VH peptide signal
<400> 33
   atgggattca gcaggatctt tctcttcctc ctgtcagtaa ctacaggtgt ccactcc 57